# EUROPEAN PATENT APPLICATION

(11) **EP 4 397 264 A1**
(43) Date of publication of application: **10.07.2024**
(21) Application number: 23219868.9
(22) Date of filing: 22.12.2023
(51) Int. Cl.: A61B 18/14, A61B 5/287, A61B 5/00, A61B 18/00

(54) **ELECTRODE ARRANGEMENT FOR PLANAR CATHETER END EFFECTOR**

(30) Priority: 28.12.2022 US 202263435652 P; 20.11.2023 US 202318514868
(71) Applicant: Biosense Webster (Israel) Ltd., 2066717 Yokneam (IL)
(72) Inventor: RODRIGUEZ SOTO, Juan M., Irvine, 92618 (US); VAN NIEKERK, Pieter E., Irvine, 92618 (US); YOO, Hoon, Irvine, 92618 (US); BASU, Shubhayu, Irvine, 92618 (US)
(74) Representative: Carpmaels & Ransford LLP

(57) **Abstract**

A catheter for electrophysiology applications includes a shaft and an end effector. The end effector is coupled to a distal end of the shaft. The end effector includes a plurality of loop members and a plurality of electrodes affixed to the plurality of loop members. Each loop member includes a corresponding pair of spines. The spines are arranged along a transverse axis that is perpendicular to a longitudinal axis defined by the shaft. The transverse axis and the longitudinal axis collectively define a plane. The electrodes are arranged asymmetrically relative to the plane.

## Description

### CROSS-REFERENCE TO RELATED APPLICATION(S)

The present application claims priority to and the benefit of U.S. Provisional Patent Application No. 63/435,652, filed December 28, 2022, the entire content of which is incorporated by reference herein.

### FIELD OF INVENTION

The present invention is directed to catheters, in particular, electrophysiology catheters for mapping and ablating patient tissue.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals may be located in tissue of an atria or a ventricle. Unwanted signals may be conducted elsewhere through heart tissue where they can initiate or continue arrhythmia.

Procedures for treating arrhythmia include surgically disrupting the origin of the signals causing the arrhythmia, as well as disrupting the conducting pathway for such signals. By mapping the electrical properties of the endocardium and the heart volume, and selectively ablating cardiac tissue by application of energy, it may be possible to cease or modify the propagation of unwanted electrical signals from one portion of the heart to another. The ablation process may destroy the unwanted electrical pathways by formation of non-conducting regions of tissue.

In this two-step procedure, which includes mapping followed by ablation, electrical activity at points in the heart may be sensed and measured by advancing a catheter containing one or more electrical sensors into the heart and acquiring data at multiple points. These data may then be utilized to select the target areas at which ablation is to be performed.

Catheters with planar end effectors can be better suited for mapping and ablating certain tissue surface topography, for example, flatter and smoother tissue surfaces, such that planar end effectors can be dragged across flatter and smoother tissue surface or maneuvered in a "sweeping" motion. Applicants recognized that there is a need to provide a catheter with a planar end effector where electrodes are arranged asymmetrically relative to the plane defined by the end effector such that the electrodes on each planar surface can better contact different locations of the tissue contact surface.

### SUMMARY OF THE DISCLOSURE

In some embodiments, a catheter for electrophysiology applications, includes a planar end effector with electrodes that are advantageously arranged asymmetrically relative to the plane defined by the end effector such that the electrodes on opposing planar surface are configured to contact different locations depending on the planar surface that is in tissue contact. The catheter includes a shaft extending along a longitudinal axis to a distal end, and an end effector coupled to the distal end of the shaft. The end effector includes a plurality of loop members and a plurality of electrodes. Each loop member of the plurality of loop members includes a corresponding pair of spines, the spines of each loop member of the plurality of loop members being arranged along a transverse axis that is perpendicular to the longitudinal axis, the transverse axis and the longitudinal axis collectively defining a plane. The plurality of electrodes are affixed to the plurality of loop members, the plurality of electrodes being arranged asymmetrically relative to the plane.

In some embodiments, the plurality of loop members include three loop members.

In some embodiments, the plurality of loop members include a pair of outer loop members and an inner loop member positioned radially inwardly of the pair of outer loop members relative to the longitudinal axis.

In some embodiments, the plurality of loop members include a pair of inner loop members and an outer loop member positioned radially outwardly of the pair of inner loop members relative to the longitudinal axis.

In some embodiments, each loop member of the plurality of loop members includes an elongate structural member.

In some embodiments, each electrode of the plurality of electrodes is affixed to the elongate structural member of the corresponding loop member of the plurality of loop members.

In some embodiments, each loop member of the plurality of loop members includes a cover disposed about the corresponding elongate structural member.

In some embodiments, each electrode of the plurality of electrodes is affixed to the cover of the corresponding loop member of the plurality of loop members.

In some embodiments, each of the spines has a rectangular cross-sectional shape.

In some embodiments, each of the spines includes a pair of flat surfaces oriented parallel to each other, the plurality of electrodes being arranged on only one of the pair of flat surfaces of each of the spines.

In some embodiments, each of the spines includes a first flat surface oriented parallel to the plane, a second flat surface oriented orthogonal to the plane, a third flat surface oriented parallel to the plane, and a fourth flat surface oriented orthogonal to the plane, the plurality of electrodes being arranged on only the first and second flat surfaces of each of the spines.

In some embodiments, each of the spines is configured with a triangular cross-sectional shape.

In some embodiments, each of the spines is configured with three flat surfaces, the plurality of electrodes being arranged on each of the three flat surfaces of each of the spines.

In some embodiments, each of the spines is configured with a circular cross-sectional shape.

In some embodiments, each of the spines is configured with a cylindrical surface, the plurality of electrodes being arranged on the cylindrical surface of each of the spines at non-uniform intervals.

In some embodiments, each of the spines is configured with at least one polygonal cross-sectional shape with a plurality of surfaces.

In some embodiments, the at least one polygonal cross-sectional shape includes at least one of a pentagon or an octagon.

In some embodiments, the plurality of surfaces are arranged symmetrically relative to the plane.

In some embodiments, the plurality of surfaces are arranged asymmetrically relative to the plane.

In some embodiments, the at least one polygonal cross-sectional shape includes a plurality of polygonal cross-sectional shapes provided along a length of the respective spine.

In some embodiments, the plurality of polygonal cross-sectional shapes includes a pentagon, a rectangle, and a triangle.

In some embodiments, each of the spines is configured with at least one multi-curved cross-sectional shape with a plurality of curves.

In some embodiments, the at least one multi-curved cross-sectional shape includes at least one of an irregular ellipse or a Reuleaux triangle.

In some embodiments, the plurality of curves are arranged symmetrically relative to the plane.

In some embodiments, the plurality of curves are arranged asymmetrically relative to the plane.

In some embodiments, each of the spines is configured with a plurality of cross-sectional shapes provided along a length of the respective spine.

In some embodiments, the plurality of cross-sectional shapes includes a square, an ellipse, and a circle.

In some embodiments, an end effector of a catheter includes a plurality of loop members and a plurality of electrodes. Each loop member of the plurality of loop members includes a corresponding pair of spines, the spines of each loop member of the plurality of loop members including at least a portion extending parallel to a longitudinal axis, the spines of each loop member of the plurality of loop members being arranged along a transverse axis that is perpendicular to the longitudinal axis, the transverse axis and the longitudinal axis collectively defining a plane. The plurality of electrodes are affixed to the plurality of loop members, the plurality of electrodes being arranged asymmetrically relative to the plane.

In some embodiments, each of the spines is configured with at least one of a rectangular cross-sectional shape, a triangular cross-sectional shape, or a circular cross-sectional shape.

In some embodiments, an end effector of a catheter includes a central loop member extending along a longitudinal axis, a first side loop member at least partially disposed on a first side of the longitudinal axis, and a second side loop member at least partially disposed on a second side of the longitudinal axis. The second side loop member, the first side loop member, and the central loop member are arranged along a transverse axis that is perpendicular to the longitudinal axis, the transverse axis and the longitudinal axis collectively defining a plane. The end effector also includes a plurality of electrodes, each of the plurality of electrodes being affixed to a corresponding one of the central loop member, the first side loop member, or the second side loop member, the plurality of electrodes being arranged asymmetrically relative to the plane.

In some embodiments, the central loop member is positioned radially inwardly of each of the first and second side loop members relative to the longitudinal axis.

In some embodiments, the central loop member is positioned radially outwardly of each of the first and second side loop members relative to the longitudinal axis.

In some embodiments, an electrode of the end effector is asymmetric relative to a plane defined by the end effector.

In some embodiments, an electrode of the end effector is asymmetric relative to a first plane that extends through the electrode and that is parallel to a plane defined by the end effector.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a front elevational view of an example of an end effector for use with the catheter of FIG. 1 and having an asymmetric arrangement of electrodes;
FIG. 2A depicts a cross-sectional view of the end effector of FIG. 2, taken along line 2A-2A in FIG. 2;
FIG. 2B depicts a cross-sectional view of another example of an end effector for use with the catheter of FIG. 1 and having an asymmetric arrangement of electrodes;
FIG. 2C depicts a cross-sectional view of another example of an end effector for use with the catheter of FIG. 1 and having an asymmetric arrangement of electrodes;
FIG. 2D depicts a cross-sectional view of another example of an end effector for use with the catheter of FIG. 1 and having an asymmetric arrangement of electrodes;
FIG. 3 depicts a front elevational view of another example of an end effector for use with the catheter of FIG. 1 and having an asymmetric arrangement of electrodes;
FIG. 3A depicts a cross-sectional view of the end effector of FIG. 3, taken along line 3A-3A in FIG. 3;
FIG. 3B depicts a cross-sectional view of another example of an end effector for use with the catheter of FIG. 1 and having an asymmetric arrangement of electrodes;
FIG. 3C depicts a cross-sectional view of another example of an end effector for use with the catheter of FIG. 1 and having an asymmetric arrangement of electrodes;
FIG. 3D depicts a cross-sectional view of another example of an end effector for use with the catheter of FIG. 1 and having an asymmetric arrangement of electrodes;
FIG. 4 depicts a front elevational view of another example of an end effector for use with the catheter of FIG. 1 and having an asymmetric arrangement of electrodes;
FIG. 4A depicts a cross-sectional view of the end effector of FIG. 4, taken along line 4A-4A in FIG. 4;
FIG. 4B depicts a cross-sectional view of another example of an end effector for use with the catheter of FIG. 1 and having an asymmetric arrangement of electrodes;
FIG. 4C depicts a cross-sectional view of another example of an end effector for use with the catheter of FIG. 1 and having an asymmetric arrangement of electrodes;
FIG. 4D depicts a cross-sectional view of another example of an end effector for use with the catheter of FIG. 1 and having an asymmetric arrangement of electrodes;
FIG. 5A is a perspective view of an end effector of FIG. 2, having the asymmetric arrangement of electrodes of FIG. 2B, with a second side in tissue contact;
FIG. 5B is a perspective view of the end effector of FIG. 5A, with a first side in tissue contact;
FIG. 5C is a perspective view of the end effector of FIG. 5A, with the first side in tissue contact;
FIG. 6A depicts a cross-sectional view of an inner loop member of another example of an end effector for use with the catheter of FIG. 1 and having an asymmetric arrangement of electrodes;
FIG. 6B depicts a perspective view of an outer loop member of the end effector of FIG. 6A and having an asymmetric arrangement of electrodes;
FIG. 6C depicts a cross-sectional view of the outer loop member of FIG. 6B, taken along line 6C-6C in FIG. 6B; and
FIG. 6D depicts a cross-sectional view of an outer loop member of another example of an end effector for use with the catheter of FIG. 1 and having an asymmetric arrangement of electrodes.

### DETAILED DESCRIPTION FOR MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81 % to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

Example end effectors are illustrated and disclosed herein which are generally planar and include multiple electrodes that can be configured for mapping and/or ablation. The end effectors can be joined to a shaft with additional catheter components to form a mapping and/or ablation catheter through processes disclosed herein and processes similar to those known by a person skilled in the pertinent art. The example end effectors illustrated herein include variations and features that are combinable to form additional end effector designs as understood by a person skilled in the pertinent art.

### I. Overview of Example of a Catheter System

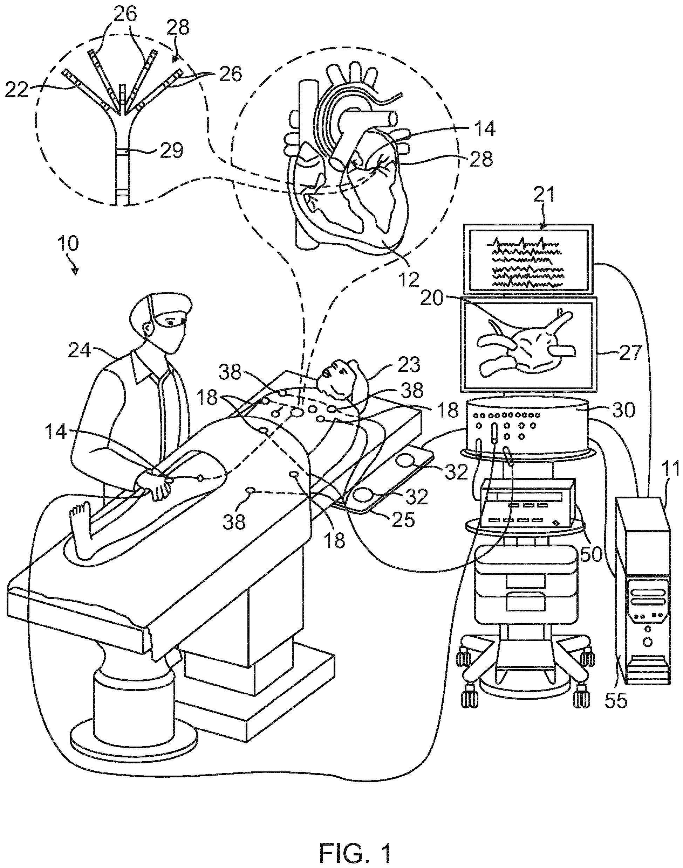

Reference is made to FIG. 1 showing an example catheter-based electrophysiology mapping and ablation system (10). System (10) includes multiple catheters, which are percutaneously inserted by physician (24) through the patient's vascular system into a chamber or vascular structure of a heart (12). Typically, a delivery sheath catheter is inserted into the left or right atrium near a desired location in heart (12). Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter (14) that is configured for sensing IEGM is illustrated herein. Physician (24) brings a distal tip (28) of catheter (14) into contact with the heart wall for sensing a target site in heart (12). For ablation, physician (24) would similarly bring a distal end of an ablation catheter to a target site for ablating.

Catheter (14) is an exemplary catheter that includes one and preferably multiple electrodes (26) optionally distributed over a plurality of splines (22) at distal tip (28) and configured to sense the IEGM signals. Catheter (14) may additionally include a position sensor (29) embedded in or near distal tip (28) for tracking position and orientation of distal tip (28). Optionally and preferably, position sensor (29) is a magnetic based position sensor including three magnetic coils for sensing three-dimensional (3D) position and orientation.

Magnetic based position sensor (29) may be operated together with a location pad (25) including a plurality of magnetic coils (32) configured to generate magnetic fields in a predefined working volume. Real time position of distal tip (28) of catheter (14) may be tracked based on magnetic fields generated with location pad (25) and sensed by magnetic based position sensor (29). Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System (10) includes one or more electrode patches (38) positioned for skin contact on patient (23) to establish location reference for location pad (25) as well as impedance-based tracking of electrodes (26). For impedance-based tracking, electrical current is directed toward electrodes (26) and sensed at electrode skin patches (38) so that the location of each electrode can be triangulated via the electrode patches (38). Details of the impedance-based location tracking technology are described in US Patent Nos. 7,536,218; 7,756,576; 7,848,787; 7,869,865; and 8,456,182.

A recorder (11) displays electrograms (21) captured with body surface ECG electrodes (18) and IEGMs captured with electrodes (26) of catheter (14). Recorder (11) may include pacing capability for pacing the heart rhythm and/or may be electrically connected to a standalone pacer.

System (10) may include an ablation energy generator (50) that is adapted to conduct ablative energy to one or more of electrodes at a distal tip of a catheter configured for ablating. Energy produced by ablation energy generator (50) may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) (30) is an interface configured to establish electrical communication between catheters, electrophysiological equipment, power supply and a workstation (55) for controlling operation of system (10). Electrophysiological equipment of system (10) may include for example, multiple catheters, location pad (25), body surface ECG electrodes (18), electrode patches (38), ablation energy generator (50), and recorder (11). Optionally and preferably, PIU (30) additionally includes processing capability for implementing real-time computations of location of the catheters and for performing ECG calculations.

Workstation (55) includes memory, processor unit with memory or storage with appropriate operating software loaded therein, and user interface capability. Workstation (55) may provide multiple functions, optionally including modeling the endocardial anatomy in three-dimensions (3D) and rendering the model or anatomical map (20) for display on a display device (27); displaying on display device (27) activation sequences (or other data) compiled from recorded electrograms (21) in representative visual indicia or imagery superimposed on the rendered anatomical map (20); displaying real-time location and orientation of multiple catheters within the heart chamber; and displaying on display device (2)7 sites of interest such as places where ablation energy has been applied. One commercial product embodying elements of the system (10) is available as the CARTO^{™} 3 System, available from Biosense Webster, Inc., 31 Technology Drive, Irvine, CA 92618.

### II. Examples of Asymmetric Electrode Arrangements for End Effectors

In some procedures, it may be desirable to provide catheter (14) with a generally planar end effector having a plurality of electrodes that are arranged asymmetrically relative to a plane defined by the end effector. It will be appreciated that such an asymmetric arrangement of electrodes may provide catheter (14) with improved mapping and/or ablating capabilities. Each of the examples of end effectors (110, 210, 310, 410, 510, 610, 710, 810, 910, 1010, 1110, 1210) described below may function in such a manner.

### A. First Example of Asymmetric Electrode Arrangement

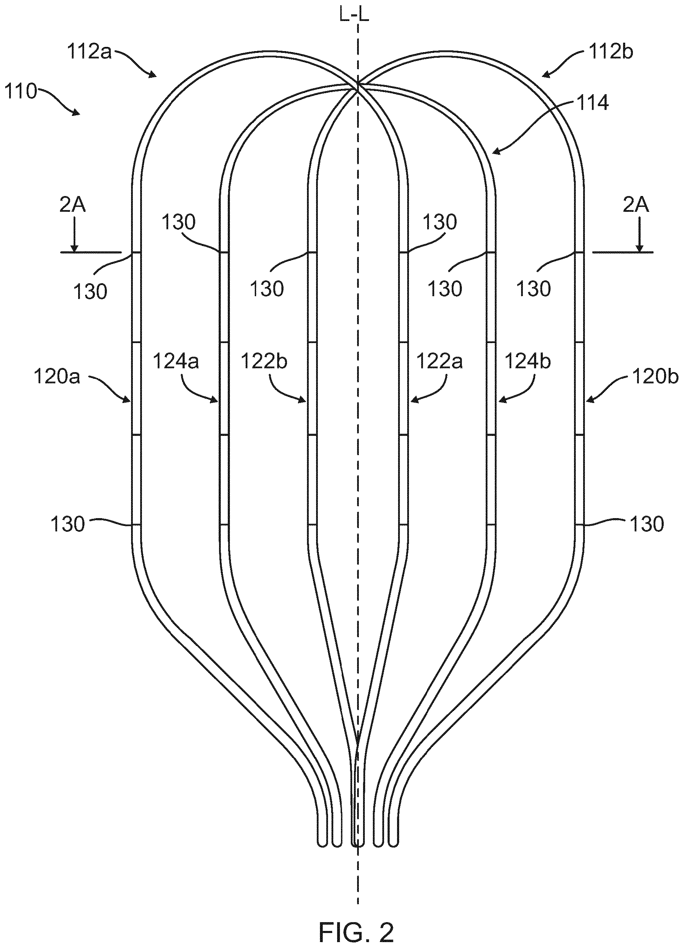

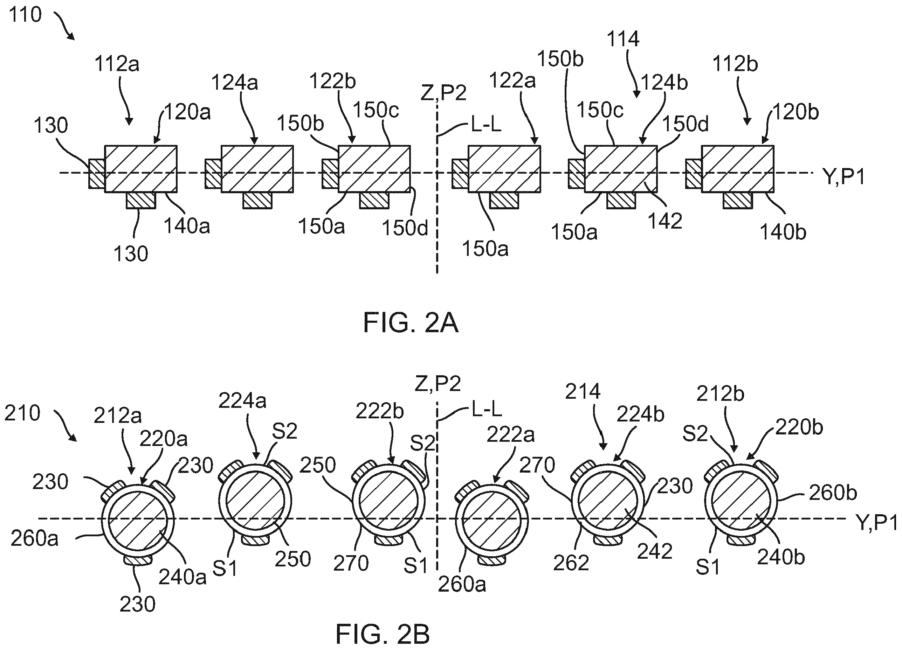

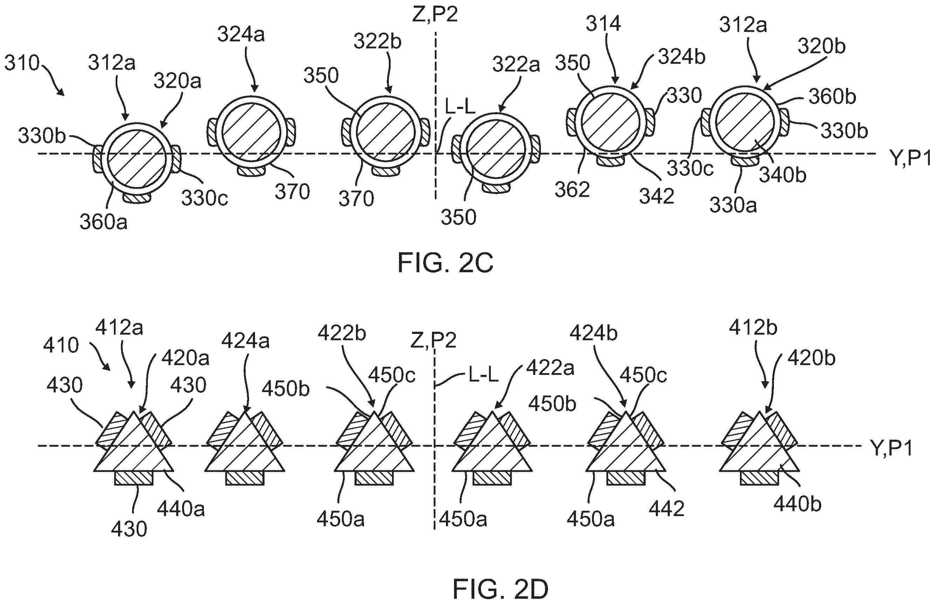

FIGS. 2-2A depict an example of an end effector (110) that may be readily incorporated into catheter (14) in place of distal tip (28). End effector (110) of the present example includes first and second outer loop members (112a, 112b) and an inner loop member (114), each extending distally from a base member or shaft (not shown), which itself extends along a longitudinal axis (L-L). Loop members (112a, 112b, 114) may also be referred to as paddles, loops, and/or electrode loop assemblies.

In the example shown, first and second outer loop members (112a, 112b) are disposed on either side of longitudinal axis (L-L), at least partially radially outwardly of third or central loop member (114). More particularly, outer loop members (112a, 112b) each include a corresponding outermost spine member (120a, 120b) and a corresponding innermost spine member (122a, 122b), while inner loop member (114) includes a pair of intermediate spine members (124a, 124b). Spine members (120a, 120b, 122a, 122b, 124a, 124b) may also be referred to as spines. In this regard, outermost spines (120a, 120b) are positioned radially outwardly of intermediate spines (124a, 124b) with respect to longitudinal axis (L-L); and innermost spines (122a, 122b) are positioned radially inwardly of intermediate spines (124a, 124b) with respect to longitudinal axis (L-L), at least within the frame of reference of FIG. 2. It will be appreciated that first outermost spine member (120a) is integral with first innermost spine member (122a) as part of first outer loop (112a) and that second outermost spine member (120b) is integral with second innermost spine member (122b) as part of second outer loop (112b). Similarly, first intermediate spine member (124a) is integral with second intermediate spine member (124b) as part of inner loop (114).

As best shown in FIG. 2A, spine members (120a, 120b, 122a, 122b, 124a, 124b) are generally arranged along a Y-axis that is orthogonal to longitudinal axis (L-L) such that end effector (110) may generally reside within a first plane (P1) defined by the Y-axis and longitudinal axis (L-L) and may therefore be generally planar. First outermost spine member (120a), first intermediate spine member (124a), and second innermost spine member (122b) are arranged on a first side of a second plane (P2) defined by longitudinal axis (L-L) and a Z-axis that is orthogonal to both the Y-axis and longitudinal axis (L-L); and second outermost spine member (120b), second intermediate spine member (124b), and first innermost spine member (122a) are arranged on a second side of the second plane (P2).

Loop members (112a, 112b, 114) also each include a corresponding plurality of sensing electrodes (130) secured to the respective spine members (120a, 120b, 122a, 122b, 124a, 124b) and arranged asymmetrically relative to the first plane (P1). Electrodes (130) may be configured to provide electrophysiology (EP) mapping, such as to identify tissue regions that should be targeted for ablation. For example, electrodes (130) may monitor electrical signals emanating from conductive endocardial tissues to pinpoint the location of aberrant conductive tissue sites that are responsible for an arrhythmia. By way of example only, electrodes (130) may be configured and operable in accordance with at least some of the teachings of U.S. Pub. No. 2020/0345262, entitled "Mapping Grid with High Density Electrode Array," published November 5, 2020, the disclosure of which is incorporated by reference herein, in its entirety.

In the example shown, each loop member (112a, 112b, 114) includes a corresponding structural member (140a, 140b, 142). In this regard, each outer loop member (112a, 112b) has a corresponding elongated structural member (140a, 140b) extending continuously through the length of the respective outer loop member (112a, 112b) to and from the base member, while inner loop member (114) has an elongated structural member (142) extending continuously through the length of inner loop member (114) to and from the base member. Structural members (140a, 140b, 142) may also be referred to as loop structural members, spine frames, or frames. Structural members (140a, 140b, 142) can each include a biocompatible metal such as stainless steel, cobalt chromium, or nitinol.

As best shown in FIG. 2A, each structural member (140a, 140b, 142) has four generally flat surfaces (150a, 150b, 150c, 150d) including a first generally flat surface (150a) oriented generally parallel to the first plane (P1), a second generally flat surface (150b) oriented generally orthogonal to the first plane (P1), a third generally flat surface (150c) oriented generally parallel to the first plane (P1), and a fourth generally flat surface (150d) oriented generally orthogonal to the first plane (P1), such that each structural member (140a, 140b, 142) of the present example has a generally rectangular cross-sectional shape. In the example shown, first and third surfaces (150a, 150c) each have a same length greater than that of second and fourth surfaces (150b, 150d). It will be appreciated that each structural member (140a, 140b, 142) may have any other suitable cross-sectional shape and/or may have any suitable number of different cross-sectional shapes that transition along a length of the structural member (140a, 140b, 142). For example, each structural member (140a, 140b, 142) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (112a, 112b, 114) may further include a corresponding cover (not shown), with the corresponding structural member (140a, 140b, 142) underlying the corresponding cover and with the corresponding plurality of electrodes (130) disposed on an outer surface of the corresponding cover (or otherwise exposed relative to the cover). Such covers may each be electrically insulative, and/or may each include a polymeric material such as polyurethane, for example.

As noted above, electrodes (130) of each loop member (112a, 112b, 114) are arranged asymmetrically relative to the first plane (P1) defined by end effector (110). In this regard, electrodes (130) of the present example are disposed only on first and second surfaces (150a, 150b) of structural members (140a, 140b, 142) of loop members (112a, 112b, 114), and are not disposed on third and four surfaces (150c, 150d) of structural members (140a, 140b, 142) of loop members (112a, 112b, 114). As shown, each electrode (130) disposed on a first surface (150a) of a structural member (140a, 140b, 142) is positioned at a same height along longitudinal axis (L-L) as a corresponding, adjacent electrode (130) disposed on the second surface (150b) of the same structural member (140a, 140b, 142), such that electrodes (130) may be arranged in adjacent groups of two. Each adjacent group of two electrodes (130) is asymmetric relative to the first plane (P1) defined by end effector (110), with the electrode (130) on first surface (150a) of each group being completely disposed on a first side of the first plane (P1) and the electrode (130) on second surface (150b) of each group spanning both the first and second sides of the first plane (P1).

While the arrangements of electrodes (130) are shown as being the same for each spine member (120a, 120b, 122a, 122b, 124a, 124b), it will be appreciated that electrodes (130) may be oriented in various different arrangements for each spine member (120a, 120b, 122a, 122b, 124a, 124b). For example, electrodes (130) may be oriented in a first arrangement on outermost spines (120a, 120b), in a second arrangement on innermost spines (122a, 122b), and in a third arrangement on intermediate spines (124a, 124b). It will be appreciated that the different arrangements of electrodes (130) may include the spacing between electrodes (130) on one spine (120a, 120b, 122a, 122b, 124a, 124b) differing from the spacing between electrodes (130) on another spine (120a, 120b, 122a, 122b, 124a, 124b). In addition, or in the alternative, the different arrangements may include the angular orientation of the electrodes (130) on one spine (120a, 120b, 122a, 122b, 124a, 124b) differing from the angular orientation of the electrodes (130) on another spine (120a, 120b, 122a, 122b, 124a, 124b). The different arrangements may also include any other way in which the electrodes (130) may be positioned and/or oriented on one spine (120a, 120b, 122a, 122b, 124a, 124b) relative to the other electrodes (130) on the same spine (120a, 120b, 122a, 122b, 124a, 124b); and how that relative positioning and/or orientation may vary from one spine (120a, 120b, 122a, 122b, 124a, 124b) to another spine (120a, 120b, 122a, 122b, 124a, 124b).

### B. Second Example of Asymmetric Electrode Arrangement

FIG. 2B depicts another example of an end effector (210) that may be readily incorporated into catheter (14) in place of distal tip (28). End effector (210) of the present example is similar to end effector (110) described above, except as otherwise described below. In this regard, end effector (210) includes first and second outer loop members (212a, 212b) and an inner loop member (214), each extending distally from a base member or shaft (not shown), which itself extends along a longitudinal axis (L-L). Outer loop members (212a, 212b) each include a corresponding outermost spine member (220a, 220b) and a corresponding innermost spine member (222a, 222b), while inner loop member (214) includes a pair of intermediate spine members (224a, 224b).

As shown, spine members (220a, 220b, 222a, 222b, 224a, 224b) are generally arranged along a Y-axis that is orthogonal to longitudinal axis (L-L) such that end effector (210) may generally reside within a first plane (P1) defined by the Y-axis and longitudinal axis (L-L) and may therefore be generally planar. First outermost spine member (220a), first intermediate spine member (224a), and second innermost spine member (222b) are arranged on a first side of a second plane (P2) defined by longitudinal axis (L-L) and a Z-axis that is orthogonal to both the Y-axis and longitudinal axis (L-L); and second outermost spine member (220b), second intermediate spine member (224b), and first innermost spine member (222a) are arranged on a second side of the second plane (P2).

Loop members (212a, 212b, 214) also each include a corresponding plurality of sensing electrodes (230) secured to the respective spine members (220a, 220b, 222a, 222b, 224a, 224b) and arranged asymmetrically relative to the first plane (P1). In the example shown, each loop member (212a, 212b, 214) includes a corresponding structural member (240a, 240b, 242), each having a generally cylindrical surface (250) such that each structural member (240a, 240b, 242) of the present example has a generally circular cross-sectional shape. It will be appreciated that each structural member (240a, 240b, 242) may have any other suitable cross-sectional shape and/or may have any suitable number of different cross-sectional shapes that transition along a length of the structural member (240a, 240b, 242). For example, each structural member (240a, 240b, 242) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (212a, 212b, 214) further includes a corresponding cover (260a, 260b, 262), with the corresponding structural member (240a, 240b, 242) underlying the corresponding cover (260a, 260b, 262) and with the corresponding plurality of electrodes (230) disposed on an outer surface (270) of the corresponding cover (260a, 260b, 262). Covers (260a, 260b, 262) may each be electrically insulative, and/or may each include a polymeric material such as polyurethane, for example.

As noted above, electrodes (230) of each loop member (212a, 212b, 214) are arranged asymmetrically relative to the first plane (P1) defined by end effector (210). In this regard, electrodes (230) of the present example are circumferentially disposed on surfaces (270) of covers (260a, 260b, 262) of loop members (212a, 212b, 214) at non-uniform intervals. As shown, each electrode (230) disposed on a surface (270) of a cover (260a, 260b, 262) is positioned at a same height along longitudinal axis (L-L) as two corresponding, adjacent electrodes (230) disposed on the surface (270) of the same cover (260a, 260b, 262), such that electrodes (230) may be arranged in adjacent groups of three. Each adjacent group of three electrodes (230) is asymmetric relative to the first plane (P1) defined by end effector (210), with one electrode (230) of each group being completely disposed on a first side of the first plane (P1) and the other two electrodes (230) of each group being completely disposed on a second side of the first plane (P1).

While the arrangements of electrodes (230) are shown as being the same for each spine member (220a, 220b, 222a, 222b, 224a, 224b), it will be appreciated that electrodes (230) may be oriented in various different arrangements for each spine member (220a, 220b, 222a, 222b, 224a, 224b). For example, electrodes (230) may be oriented in a first arrangement on outermost spines (220a, 220b), in a second arrangement on innermost spines (222a, 222b), and in a third arrangement on intermediate spines (224a, 224b). It will be appreciated that the different arrangements of electrodes (230) may include the spacing between electrodes (230) on one spine (220a, 220b, 222a, 222b, 224a, 224b) differing from the spacing between electrodes (230) on another spine (220a, 220b, 222a, 222b, 224a, 224b). In addition, or in the alternative, the different arrangements may include the angular orientation of the electrodes (230) on one spine (220a, 220b, 222a, 222b, 224a, 224b) differing from the angular orientation of the electrodes (230) on another spine (220a, 220b, 222a, 222b, 224a, 224b). The different arrangements may also include any other way in which the electrodes (230) may be positioned and/or oriented on one spine (220a, 220b, 222a, 222b, 224a, 224b) relative to the other electrodes (230) on the same spine (220a, 220b, 222a, 222b, 224a, 224b); and how that relative positioning and/or orientation may vary from one spine (220a, 220b, 222a, 222b, 224a, 224b) to another spine (220a, 220b, 222a, 222b, 224a, 224b).

### C. Third Example of Asymmetric Electrode Arrangement

FIG. 2C depicts another example of an end effector (310) that may be readily incorporated into catheter (14) in place of distal tip (28). End effector (310) of the present example is similar to end effector (110) described above, except as otherwise described below. In this regard, end effector (310) includes first and second outer loop members (312a, 312b) and an inner loop member (314), each extending distally from a base member or shaft (not shown), which itself extends along a longitudinal axis (L-L). Outer loop members (312a, 312b) each include a corresponding outermost spine member (320a, 320b) and a corresponding innermost spine member (322a, 322b), while inner loop member (314) includes a pair of intermediate spine members (324a, 324b).

As shown, spine members (320a, 320b, 322a, 322b, 324a, 324b) are generally arranged along a Y-axis that is orthogonal to longitudinal axis (L-L) such that end effector (310) may generally reside within a first plane (P1) defined by the Y-axis and longitudinal axis (L-L) and may therefore be generally planar. First outermost spine member (320a), first intermediate spine member (324a), and second innermost spine member (322b) are arranged on a first side of a second plane (P2) defined by longitudinal axis (L-L) and a Z-axis that is orthogonal to both the Y-axis and longitudinal axis (L-L); and second outermost spine member (320b), second intermediate spine member (324b), and first innermost spine member (322a) are arranged on a second side of the second plane (P2).

Loop members (312a, 312b, 314) also each include a corresponding plurality of sensing electrodes (330) secured to the respective spine members (320a, 320b, 322a, 322b, 324a, 324b) and arranged asymmetrically relative to the first plane (P1). In the example shown, each loop member (312a, 312b, 314) includes a corresponding structural member (340a, 340b, 342), each having a generally cylindrical surface (350) such that each structural member (340a, 340b, 342) of the present example has a generally circular cross-sectional shape, each having a generally cylindrical surface (350) such that each structural member (340a, 340b, 342) of the present example has a generally circular cross-sectional shape. It will be appreciated that each structural member (340a, 340b, 342) may have any other suitable cross-sectional shape and/or may have any suitable number of different cross-sectional shapes that transition along a length of the structural member (340a, 340b, 342). For example, each structural member (340a, 340b, 342) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (312a, 312b, 314) further includes a corresponding cover (360a, 360b, 362), with the corresponding structural member (340a, 340b, 342) underlying the corresponding cover (360a, 360b, 362) and with the corresponding plurality of electrodes (330) disposed on an outer surface (370) of the corresponding cover (360a, 360b, 362). Covers (360a, 360b, 362) may each be electrically insulative, and/or may each include a polymeric material such as polyurethane, for example.

As noted above, electrodes (330) of each loop member (312a, 312b, 314) are arranged asymmetrically relative to the first plane (P1) defined by end effector (310). In this regard, electrodes (330) of the present example are circumferentially disposed on surfaces (370) of covers (360a, 360b, 362) of loop members (312a, 312b, 314) at non-uniform intervals. As shown, each electrode (330) disposed on a surface (370) of a cover (360a, 360b, 362) is positioned at a same height along longitudinal axis (L-L) as two corresponding, adjacent electrodes (330) disposed on the surface (370) of the same cover (360a, 360b, 362), such that electrodes (330) may be arranged in adjacent groups of three. Each adjacent group of three electrodes (330) is asymmetric relative to the first plane (P1) defined by end effector (310), with one electrode (330) of each group being at least partially disposed on a first side of the first plane (P1) and the other two electrodes (330) of each group being at least partially disposed on a second side of the first plane (P1).

While the arrangements of electrodes (330) are shown as being the same for each spine member (320a, 320b, 322a, 322b, 324a, 324b), it will be appreciated that electrodes (330) may be oriented in various different arrangements for each spine member (320a, 320b, 322a, 322b, 324a, 324b). For example, electrodes (330) may be oriented in a first arrangement on outermost spines (320a, 320b), in a second arrangement on innermost spines (322a, 322b), and in a third arrangement on intermediate spines (324a, 324b). It will be appreciated that the different arrangements of electrodes (330) may include the spacing between electrodes (330) on one spine (320a, 320b, 322a, 322b, 324a, 324b) differing from the spacing between electrodes (330) on another spine (320a, 320b, 322a, 322b, 324a, 324b). In addition, or in the alternative, the different arrangements may include the angular orientation of the electrodes (330) on one spine (320a, 320b, 322a, 322b, 324a, 324b) differing from the angular orientation of the electrodes (330) on another spine (320a, 320b, 322a, 322b, 324a, 324b). The different arrangements may also include any other way in which the electrodes (330) may be positioned and/or oriented on one spine (320a, 320b, 322a, 322b, 324a, 324b) relative to the other electrodes (330) on the same spine (320a, 320b, 322a, 322b, 324a, 324b); and how that relative positioning and/or orientation may vary from one spine (320a, 320b, 322a, 322b, 324a, 324b) to another spine (320a, 320b, 322a, 322b, 324a, 324b).

### D. Fourth Example of Asymmetric Electrode Arrangement

FIG. 2D depicts another example of an end effector (410) that may be readily incorporated into catheter (14) in place of distal tip (28). End effector (410) of the present example is similar to end effector (110) described above, except as otherwise described below. In this regard, end effector (410) includes first and second outer loop members (412a, 412b) and an inner loop member (414), each extending distally from a base member or shaft (not shown), which itself extends along a longitudinal axis (L-L). Outer loop members (412a, 412b) each include a corresponding outermost spine member (420a, 420b) and a corresponding innermost spine member (422a, 422b), while inner loop member (414) includes a pair of intermediate spine members (424a, 424b).

As shown, spine members (420a, 420b, 422a, 422b, 424a, 424b) are generally arranged along a Y-axis that is orthogonal to longitudinal axis (L-L) such that end effector (410) may generally reside within a first plane (P1) defined by the Y-axis and longitudinal axis (L-L) and may therefore be generally planar. First outermost spine member (420a), first intermediate spine member (424a), and second innermost spine member (422b) are arranged on a first side of a second plane (P2) defined by longitudinal axis (L-L) and a Z-axis that is orthogonal to both the Y-axis and longitudinal axis (L-L); and second outermost spine member (420b), second intermediate spine member (424b), and first innermost spine member (422a) are arranged on a second side of the second plane (P2).

Loop members (412a, 412b, 414) also each include a corresponding plurality of sensing electrodes (430) secured to the respective spine members (420a, 420b, 422a, 422b, 424a, 424b) and arranged asymmetrically relative to the first plane (P1). In the example shown, each loop member (412a, 412b, 414) includes a corresponding structural member (440a, 440b, 442), each having three generally flat surfaces (450a, 450b, 450c) including a first generally flat surface (450a) oriented generally parallel to the first plane (P1), a second generally flat surface (450b) oriented generally obliquely to the first plane (P1), and a third generally flat surface (450c) oriented generally obliquely to the first plane (P1), such that each structural member (440a, 440b, 442) of the present example has a generally triangular cross-sectional shape. In the example shown, first, second, and third surfaces (450a, 450b, 450c) each have a same length. It will be appreciated that each structural member (440a, 440b, 442) may have any other suitable cross-sectional shape and/or may have any suitable number of different cross-sectional shapes that transition along a length of the structural member (440a, 440b, 442). For example, each structural member (440a, 440b, 442) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (412a, 412b, 414) may further include a corresponding cover (not shown), with the corresponding structural member (440a, 440b, 442) underlying the corresponding cover and with the corresponding plurality of electrodes (430) disposed on an outer surface of the corresponding cover (or otherwise exposed relative to the cover). Such covers may each be electrically insulative, and/or may each include a polymeric material such as polyurethane, for example.

As noted above, electrodes (430) of each loop member (412a, 412b, 414) are arranged asymmetrically relative to the first plane (P1) defined by end effector (410). In this regard, electrodes (430) of the present example are disposed on each surface (450a, 450b, 450c) of structural members (440a, 440b, 442) of loop members (412a, 412b, 414). As shown, each electrode (430) disposed on a first surface (450a) of a structural member (440a, 440b, 442) is positioned at a same height along longitudinal axis (L-L) as two corresponding, adjacent electrodes (430) disposed on the second and third surfaces (450b, 450c) of the same structural member (440a, 440b, 442), such that electrodes (430) may be arranged in adjacent groups of three. Each adjacent group of three electrodes (430) is asymmetric relative to the first plane (P1) defined by end effector (410), with the electrode (430) on first surface (450a) of each group being completely disposed on a first side of the first plane (P1) and the other two electrodes (430) on second and third surfaces (450b, 450c) of each group being at least partially disposed on a second side of the first plane (P1).

While the arrangements of electrodes (430) are shown as being the same for each spine member (420a, 420b, 422a, 422b, 424a, 424b), it will be appreciated that electrodes (430) may be oriented in various different arrangements for each spine member (420a, 420b, 422a, 422b, 424a, 424b). For example, electrodes (430) may be oriented in a first arrangement on outermost spines (420a, 420b), in a second arrangement on innermost spines (422a, 422b), and in a third arrangement on intermediate spines (424a, 424b). It will be appreciated that the different arrangements of electrodes (430) may include the spacing between electrodes (430) on one spine (420a, 420b, 422a, 422b, 424a, 424b) differing from the spacing between electrodes (430) on another spine (420a, 420b, 422a, 422b, 424a, 424b). In addition, or in the alternative, the different arrangements may include the angular orientation of the electrodes (430) on one spine (420a, 420b, 422a, 422b, 424a, 424b) differing from the angular orientation of the electrodes (430) on another spine (420a, 420b, 422a, 422b, 424a, 424b). The different arrangements may also include any other way in which the electrodes (430) may be positioned and/or oriented on one spine (420a, 420b, 422a, 422b, 424a, 424b) relative to the other electrodes (430) on the same spine (420a, 420b, 422a, 422b, 424a, 424b); and how that relative positioning and/or orientation may vary from one spine (420a, 420b, 422a, 422b, 424a, 424b) to another spine (420a, 420b, 422a, 422b, 424a, 424b).

### E. Fifth Example of Asymmetric Electrode Arrangement

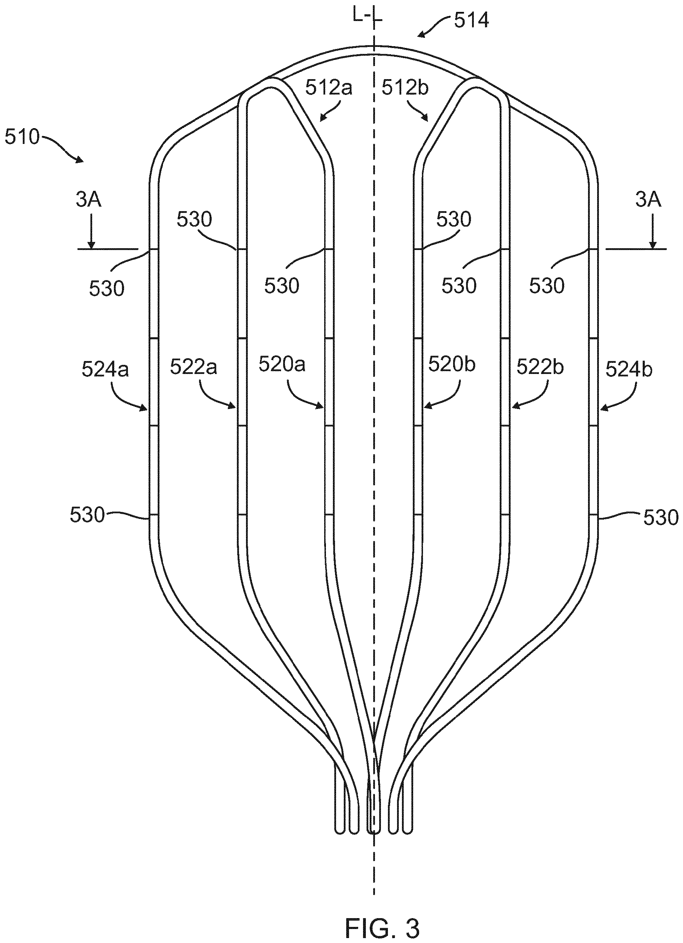

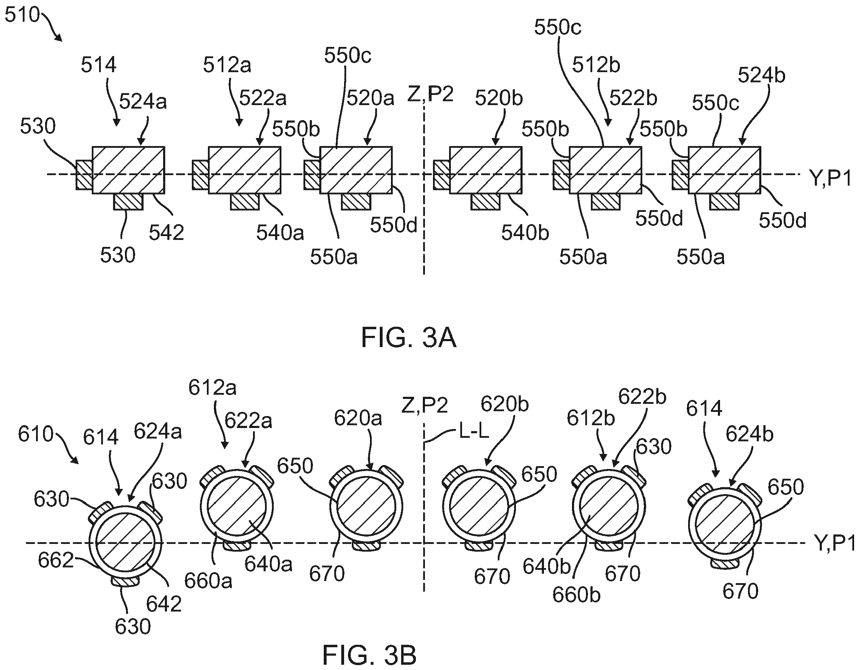

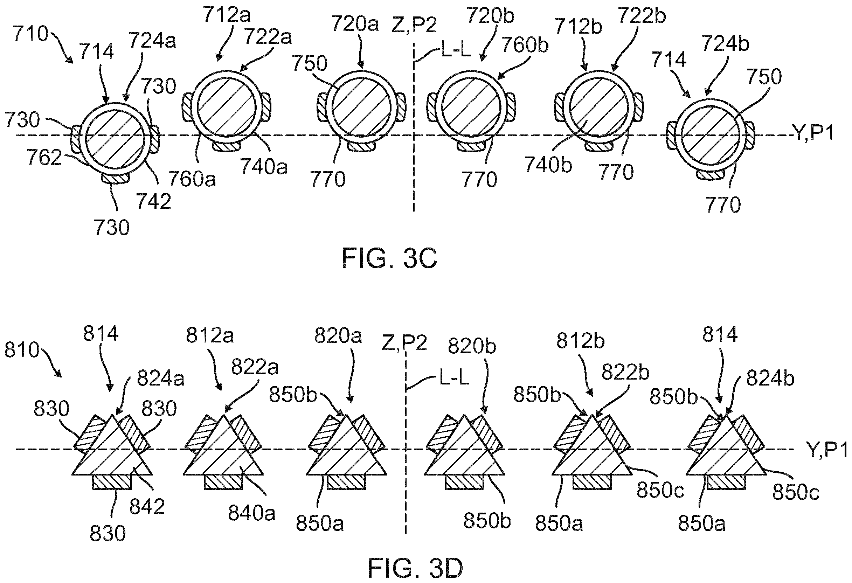

FIGS. 3-3A depict another example of an end effector (510) that may be readily incorporated into catheter (14) in place of distal tip (28). End effector (510) of the present example includes first and second inner loop members (512a, 512b) and an outer loop member (514), each extending distally from a base member or shaft (not shown), which itself extends along a longitudinal axis (L-L). Loop members (512a, 512b, 514) may also be referred to as paddles, loops, and/or electrode loop assemblies.

In the example shown, first and second inner loop members (512a, 512b) are disposed on either side of longitudinal axis (L-L), radially inwardly of third or outer loop member (514). More particularly, inner loop members (512a, 512b) each include a corresponding innermost spine member (520a, 520b) and a corresponding intermediate spine member (522a, 522b), while outer loop member (514) includes a pair of outermost spine members (524a, 524b). Spine members (520a, 520b, 522a, 522b, 524a, 524b) may also be referred to as spines. In this regard, innermost spines (520a, 520b) are positioned radially inwardly of intermediate spines (522a, 522b) with respect to longitudinal axis (L-L), and outermost spines (524a, 524b) are positioned radially outwardly of intermediate spines (522a, 522b) with respect to longitudinal axis (L-L), at least within the frame of reference of FIG. 3. It will be appreciated that first innermost spine member (520a) is integral with first intermediate spine member (522a) as part of first inner loop (512a) and that second innermost spine member (520b) is integral with second intermediate spine member (522b) as part of second inner loop (512b). Similarly, first outermost spine member (524a) is integral with second outermost spine member (524b) as part of outer loop (514).

As best shown in FIG. 3A, spine members (520a, 520b, 522a, 522b, 524a, 524b) are generally arranged along a Y-axis that is orthogonal to longitudinal axis (L-L) such that end effector (510) may generally reside within a first plane (P1) defined by the Y-axis and longitudinal axis (L-L) and may therefore be generally planar. First outermost spine member (524a), first intermediate spine member (522a), and first innermost spine member (520a) are arranged on a first side of a second plane (P2) defined by longitudinal axis (L-L) and a Z-axis that is orthogonal to both the Y-axis and longitudinal axis (L-L); and second outermost spine member (524b), second intermediate spine member (522b), and second innermost spine member (520b) are arranged on a second side of the second plane (P2).

Loop members (512a, 512b, 514) also each include a corresponding plurality of sensing electrodes (530) secured to the respective spine members (520a, 520b, 522a, 522b, 524a, 524b) and arranged asymmetrically relative to the first plane (P1). Electrodes (530) may be configured to provide electrophysiology (EP) mapping, such as to identify tissue regions that should be targeted for ablation. For example, electrodes (530) may monitor electrical signals emanating from conductive endocardial tissues to pinpoint the location of aberrant conductive tissue sites that are responsible for an arrhythmia. By way of example only, electrodes (530) may be configured and operable in accordance with at least some of the teachings of U.S. Pub. No. 2020/0345262, entitled "Mapping Grid with High Density Electrode Array," published November 5, 2020, the disclosure of which is incorporated by reference herein, in its entirety.

In the example shown, each loop member (512a, 512b, 514) includes a corresponding structural member (540a, 540b, 542). In this regard, each inner loop member (512a, 512b) has a corresponding elongated structural member (540a, 540b) extending continuously through the length of the respective inner loop member (512a, 512b) to and from the base member, while outer loop member (514) has an elongated structural member (542) extending continuously through the length of outer loop member (514) to and from the base member. Structural members (540a, 540b, 542) may also be referred to as loop structural members, spine frames, or frames. Structural members (540a, 540b, 542) can each include a biocompatible metal such as stainless steel, cobalt chromium, or nitinol.

As best shown in FIG. 3A, each structural member (540a, 540b, 542) has four generally flat surfaces (550a, 550b, 550c, 550d) including a first generally flat surface (550a) oriented generally parallel to the first plane (P1), a second generally flat surface (550b) oriented generally orthogonal to the first plane (P1), a third generally flat surface (550c) oriented generally parallel to the first plane (P1), and a fourth generally flat surface (550d) oriented generally orthogonal to the first plane (P1), such that each structural member (540a, 540b, 542) of the present example has a generally rectangular cross-sectional shape. In the example shown, first and third surfaces (550a, 550c) each have a same length greater than that of second and fourth surfaces (550b, 550d). It will be appreciated that each structural member (540a, 540b, 542) may have any other suitable cross-sectional shape and/or may have any suitable number of different cross-sectional shapes that transition along a length of the structural member (540a, 540b, 542). For example, each structural member (540a, 540b, 542) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (512a, 512b, 514) may further include a corresponding cover (not shown), with the corresponding structural member (540a, 540b, 542) underlying the corresponding cover and with the corresponding plurality of electrodes (530) disposed on an outer surface of the corresponding cover (or otherwise exposed relative to the cover). Such covers may each be electrically insulative, and/or may each include a polymeric material such as polyurethane, for example.

As noted above, electrodes (530) of each loop member (512a, 512b, 514) are arranged asymmetrically relative to the first plane (P1) defined by end effector (510). In this regard, electrodes (530) of the present example are disposed only on first and second surfaces (550a, 550b) of structural members (540a, 540b, 542) of loop members (512a, 512b, 514), and are not disposed on third and four surfaces (550c, 550d) of structural members (540a, 540b, 542) of loop members (512a, 512b, 514). As shown, each electrode (530) disposed on a first surface (550a) of a structural member (540a, 540b, 542) is positioned at a same height along longitudinal axis (L-L) as a corresponding, adjacent electrode (530) disposed on the second surface (550b) of the same structural member (540a, 540b, 542), such that electrodes (530) may be arranged in adjacent groups of two. Each adjacent group of two electrodes (530) is asymmetric relative to the first plane (P1) defined by end effector (510), with the electrode (530) on first surface (550a) of each group being completely disposed on a first side of the first plane (P1) and the electrode (530) on second surface (550b) of each group spanning both the first and second sides of the first plane (P1).

While the arrangements of electrodes (530) are shown as being the same for each spine member (520a, 520b, 522a, 522b, 524a, 524b), it will be appreciated that electrodes (530) may be oriented in various different arrangements for each spine member (520a, 520b, 522a, 522b, 524a, 524b). For example, electrodes (530) may be oriented in a first arrangement on outermost spines (524a, 524b), in a second arrangement on innermost spines (520a, 520b), and in a third arrangement on intermediate spines (522a, 522b). It will be appreciated that the different arrangements of electrodes (530) may include the spacing between electrodes (530) on one spine (520a, 520b, 522a, 522b, 524a, 524b) differing from the spacing between electrodes (530) on another spine (520a, 520b, 522a, 522b, 524a, 524b). In addition, or in the alternative, the different arrangements may include the angular orientation of the electrodes (530) on one spine (520a, 520b, 522a, 522b, 524a, 524b) differing from the angular orientation of the electrodes (530) on another spine (520a, 520b, 522a, 522b, 524a, 524b). The different arrangements may also include any other way in which the electrodes (530) may be positioned and/or oriented on one spine (520a, 520b, 522a, 522b, 524a, 524b) relative to the other electrodes (530) on the same spine (520a, 520b, 522a, 522b, 524a, 524b); and how that relative positioning and/or orientation may vary from one spine (520a, 520b, 522a, 522b, 524a, 524b) to another spine (520a, 520b, 522a, 522b, 524a, 524b).

### F. Sixth Example of Asymmetric Electrode Arrangement

FIG. 3B depicts another example of an end effector (610) that may be readily incorporated into catheter (14) in place of distal tip (28). End effector (610) of the present example is similar to end effector (510) described above, except as otherwise described below. In this regard, end effector (610) includes first and second inner loop members (612a, 612b) and an outer loop member (614), each extending distally from a base member or shaft (not shown), which itself extends along a longitudinal axis (L-L). Inner loop members (612a, 612b) each include a corresponding innermost spine member (620a, 620b) and a corresponding intermediate spine member (622a, 622b), while outer loop member (614) includes a pair of outermost spine members (624a, 624b).

As shown, spine members (620a, 620b, 622a, 622b, 624a, 624b) are generally arranged along a Y-axis that is orthogonal to longitudinal axis (L-L) such that end effector (610) may generally reside within a first plane (P1) defined by the Y-axis and longitudinal axis (L-L) and may therefore be generally planar. First outermost spine member (624a), first intermediate spine member (622a), and first innermost spine member (620a) are arranged on a first side of a second plane (P2) defined by longitudinal axis (L-L) and a Z-axis that is orthogonal to both the Y-axis and longitudinal axis (L-L); and second outermost spine member (624b), second intermediate spine member (622b), and second innermost spine member (620b) are arranged on a second side of the second plane (P2).

Loop members (612a, 612b, 614) also each include a corresponding plurality of sensing electrodes (630) secured to the respective spine members (620a, 620b, 622a, 622b, 624a, 624b) and arranged asymmetrically relative to the first plane (P1). In the example shown, each loop member (612a, 612b, 614) includes a corresponding structural member (640a, 640b, 642), each having a generally cylindrical surface (650) such that each structural member (640a, 640b, 642) of the present example has a generally circular cross-sectional shape. It will be appreciated that each structural member (640a, 640b, 642) may have any other suitable cross-sectional shape and/or may have any suitable number of different cross-sectional shapes that transition along a length of the structural member (640a, 640b, 642). For example, each structural member (640a, 640b, 642) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (612a, 612b, 614) further includes a corresponding cover (660a, 660b, 662), with the corresponding structural member (640a, 640b, 642) underlying the corresponding cover (660a, 660b, 662) and with the corresponding plurality of electrodes (630) disposed on an outer surface (670) of the corresponding cover (660a, 660b, 662). Covers (660a, 660b, 662) may each be electrically insulative, and/or may each include a polymeric material such as polyurethane, for example.

As noted above, electrodes (630) of each loop member (612a, 612b, 614) are arranged asymmetrically relative to the first plane (P1) defined by end effector (610). In this regard, electrodes (630) of the present example are circumferentially disposed on surfaces (670) of covers (660a, 660b, 662) of loop members (612a, 612b, 614) at non-uniform intervals. As shown, each electrode (630) disposed on a surface (670) of a cover (660a, 660b, 662) is positioned at a same height along longitudinal axis (L-L) as two corresponding, adjacent electrodes (630) disposed on the surface (670) of the same cover (660a, 660b, 662), such that electrodes (630) may be arranged in adjacent groups of three. Each adjacent group of three electrodes (630) is asymmetric relative to the first plane (P1) defined by end effector (610), with one electrode (630) of each group being at least partially disposed on a first side of the first plane (P1) and the other two electrodes (630) of each group being completely disposed on a second side of the first plane (P1).

While the arrangements of electrodes (630) are shown as being the same for each spine member (620a, 620b, 622a, 622b, 624a, 624b), it will be appreciated that electrodes (630) may be oriented in various different arrangements for each spine member (620a, 620b, 622a, 622b, 624a, 624b). For example, electrodes (630) may be oriented in a first arrangement on outermost spines (624a, 624b), in a second arrangement on innermost spines (620a, 620b), and in a third arrangement on intermediate spines (622a, 622b). It will be appreciated that the different arrangements of electrodes (630) may include the spacing between electrodes (630) on one spine (620a, 620b, 622a, 622b, 624a, 624b) differing from the spacing between electrodes (630) on another spine (620a, 620b, 622a, 622b, 624a, 624b). In addition, or in the alternative, the different arrangements may include the angular orientation of the electrodes (630) on one spine (620a, 620b, 622a, 622b, 624a, 624b) differing from the angular orientation of the electrodes (630) on another spine (620a, 620b, 622a, 622b, 624a, 624b). The different arrangements may also include any other way in which the electrodes (630) may be positioned and/or oriented on one spine (620a, 620b, 622a, 622b, 624a, 624b) relative to the other electrodes (630) on the same spine (620a, 620b, 622a, 622b, 624a, 624b); and how that relative positioning and/or orientation may vary from one spine (620a, 620b, 622a, 622b, 624a, 624b) to another spine (620a, 620b, 622a, 622b, 624a, 624b).

### G. Seventh Example of Asymmetric Electrode Arrangement

FIG. 3C depicts another example of an end effector (710) that may be readily incorporated into catheter (14) in place of distal tip (28). End effector (710) of the present example is similar to end effector (510) described above, except as otherwise described below. In this regard, end effector (710) includes first and second inner loop members (712a, 712b) and an outer loop member (714), each extending distally from a base member or shaft (not shown), which itself extends along a longitudinal axis (L-L). Inner loop members (712a, 712b) each include a corresponding innermost spine member (720a, 720b) and a corresponding intermediate spine member (722a, 722b), while outer loop member (714) includes a pair of outermost spine members (724a, 724b).

As shown, spine members (720a, 720b, 722a, 722b, 724a, 724b) are generally arranged along a Y-axis that is orthogonal to longitudinal axis (L-L) such that end effector (710) may generally reside within a first plane (P1) defined by the Y-axis and longitudinal axis (L-L) and may therefore be generally planar. First outermost spine member (724a), first intermediate spine member (722a), and first innermost spine member (720a) are arranged on a first side of a second plane (P2) defined by longitudinal axis (L-L) and a Z-axis that is orthogonal to both the Y-axis and longitudinal axis (L-L); and second outermost spine member (724b), second intermediate spine member (722b), and second innermost spine member (720b) are arranged on a second side of the second plane (P2).

Loop members (712a, 712b, 714) also each include a corresponding plurality of sensing electrodes (730) secured to the respective spine members (720a, 720b, 722a, 722b, 724a, 724b) and arranged asymmetrically relative to the first plane (P1). In the example shown, each loop member (712a, 712b, 714) includes a corresponding structural member (740a, 740b, 742), each having a generally cylindrical surface (750) such that each structural member (740a, 740b, 742) of the present example has a generally circular cross-sectional shape. It will be appreciated that each structural member (740a, 740b, 742) may have any other suitable cross-sectional shape and/or may have any suitable number of different cross-sectional shapes that transition along a length of the structural member (740a, 740b, 742). For example, each structural member (740a, 740b, 742) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (712a, 712b, 714) further includes a corresponding cover (760a, 760b, 762), with the corresponding structural member (740a, 740b, 742) underlying the corresponding cover (760a, 760b, 762) and with the corresponding plurality of electrodes (730) disposed on an outer surface (770) of the corresponding cover (760a, 760b, 762). Covers (760a, 760b, 762) may each be electrically insulative, and/or may each include a polymeric material such as polyurethane, for example.

As noted above, electrodes (730) of each loop member (712a, 712b, 714) are arranged asymmetrically relative to the first plane (P1) defined by end effector (710). In this regard, electrodes (730) of the present example are circumferentially disposed on surfaces (770) of covers (760a, 760b, 762) of loop members (712a, 712b, 714) at non-uniform intervals. As shown, each electrode (730) disposed on a surface (770) of a cover (760a, 760b, 762) is positioned at a same height along longitudinal axis (L-L) as two corresponding, adjacent electrodes (730) disposed on the surface (770) of the same cover (760a, 760b, 762), such that electrodes (730) may be arranged in adjacent groups of three. Each adjacent group of three electrodes (730) is asymmetric relative to the first plane (P1) defined by end effector (710), with one electrode (730) of each group being at least partially disposed on a first side of the first plane (P1) and the other two electrodes (730) of each group being at least partially disposed on a second side of the first plane (P1).

While the arrangements of electrodes (730) are shown as being the same for each spine member (720a, 720b, 722a, 722b, 724a, 724b), it will be appreciated that electrodes (730) may be oriented in various different arrangements for each spine member (720a, 720b, 722a, 722b, 724a, 724b). For example, electrodes (730) may be oriented in a first arrangement on outermost spines (724a, 724b), in a second arrangement on innermost spines (720a, 720b), and in a third arrangement on intermediate spines (722a, 722b). It will be appreciated that the different arrangements of electrodes (730) may include the spacing between electrodes (730) on one spine (720a, 720b, 722a, 722b, 724a, 724b) differing from the spacing between electrodes (730) on another spine (720a, 720b, 722a, 722b, 724a, 724b). In addition, or in the alternative, the different arrangements may include the angular orientation of the electrodes (730) on one spine (720a, 720b, 722a, 722b, 724a, 724b) differing from the angular orientation of the electrodes (730) on another spine (720a, 720b, 722a, 722b, 724a, 724b). The different arrangements may also include any other way in which the electrodes (730) may be positioned and/or oriented on one spine (720a, 720b, 722a, 722b, 724a, 724b) relative to the other electrodes (730) on the same spine (720a, 720b, 722a, 722b, 724a, 724b); and how that relative positioning and/or orientation may vary from one spine (720a, 720b, 722a, 722b, 724a, 724b) to another spine (720a, 720b, 722a, 722b, 724a, 724b).

### H. Eighth Example of Asymmetric Electrode Arrangement

FIG. 3D depicts another example of an end effector (810) that may be readily incorporated into catheter (14) in place of distal tip (28). End effector (810) of the present example is similar to end effector (510) described above, except as otherwise described below. In this regard, end effector (810) includes first and second inner loop members (812a, 812b) and an outer loop member (814), each extending distally from a base member or shaft (not shown), which itself extends along a longitudinal axis (L-L). Inner loop members (812a, 812b) each include a corresponding innermost spine member (820a, 820b) and a corresponding intermediate spine member (822a, 822b), while outer loop member (814) includes a pair of outermost spine members (824a, 824b).

As shown, spine members (820a, 820b, 822a, 822b, 824a, 824b) are generally arranged along a Y-axis that is orthogonal to longitudinal axis (L-L) such that end effector (810) may generally reside within a first plane (P1) defined by the Y-axis and longitudinal axis (L-L) and may therefore be generally planar. First outermost spine member (824a), first intermediate spine member (822a), and first innermost spine member (820a) are arranged on a first side of a second plane (P2) defined by longitudinal axis (L-L) and a Z-axis that is orthogonal to both the Y-axis and longitudinal axis (L-L); and second outermost spine member (824b), second intermediate spine member (822b), and second innermost spine member (820b) are arranged on a second side of the second plane (P2).

Loop members (812a, 812b, 814) also each include a corresponding plurality of sensing electrodes (830) secured to the respective spine members (820a, 820b, 822a, 822b, 824a, 824b) and arranged asymmetrically relative to the first plane (P1). In the example shown, each loop member (812a, 812b, 814) includes a corresponding structural member (840a, 840b, 842), each having three generally flat surfaces (850a, 850b, 850c) including a first generally flat surface (850a) oriented generally parallel to the first plane (P1), a second generally flat surface (850b) oriented generally obliquely to the first plane (P1), and a third generally flat surface (850c) oriented generally obliquely to the first plane (P1), such that each structural member (840a, 840b, 842) of the present example has a generally triangular cross-sectional shape. In the example shown, first, second, and third surfaces (850a, 850b, 850c) each have a same length. It will be appreciated that each structural member (840a, 840b, 842) may have any other suitable cross-sectional shape and/or may have any suitable number of different cross-sectional shapes that transition along a length of the structural member (840a, 840b, 842). For example, each structural member (840a, 840b, 842) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (812a, 812b, 814) may further include a corresponding cover (not shown), with the corresponding structural member (840a, 840b, 842) underlying the corresponding cover and with the corresponding plurality of electrodes (830) disposed on an outer surface of the corresponding cover (or otherwise exposed relative to the cover). Such covers may each be electrically insulative, and/or may each include a polymeric material such as polyurethane, for example.

As noted above, electrodes (830) of each loop member (812a, 812b, 814) are arranged asymmetrically relative to the first plane (P1) defined by end effector (810). In this regard, electrodes (830) of the present example are disposed on each surface (850a, 850b, 850c) of structural members (840a, 840b, 842) of loop members (812a, 812b, 814). As shown, each electrode (830) disposed on a first surface (850a) of a structural member (840a, 840b, 842) is positioned at a same height along longitudinal axis (L-L) as two corresponding, adjacent electrodes (830) disposed on the second and third surfaces (850b, 850c) of the same structural member (840a, 840b, 842), such that electrodes (830) may be arranged in adjacent groups of three. Each adjacent group of three electrodes (830) is asymmetric relative to the first plane (P1) defined by end effector (810), with the electrode (830) on first surface (850a) of each group being completely disposed on a first side of the first plane (P1) and the other two electrodes (830) on second and third surfaces (850b, 850c) of each group being at least partially disposed on a second side of the first plane (P1).

While the arrangements of electrodes (830) are shown as being the same for each spine member (820a, 820b, 822a, 822b, 824a, 824b), it will be appreciated that electrodes (830) may be oriented in various different arrangements for each spine member (820a, 820b, 822a, 822b, 824a, 824b). For example, electrodes (830) may be oriented in a first arrangement on outermost spines (824a, 824b), in a second arrangement on innermost spines (820a, 820b), and in a third arrangement on intermediate spines (822a, 822b). It will be appreciated that the different arrangements of electrodes (830) may include the spacing between electrodes (830) on one spine (820a, 820b, 822a, 822b, 824a, 824b) differing from the spacing between electrodes (830) on another spine (820a, 820b, 822a, 822b, 824a, 824b). In addition, or in the alternative, the different arrangements may include the angular orientation of the electrodes (830) on one spine (820a, 820b, 822a, 822b, 824a, 824b) differing from the angular orientation of the electrodes (830) on another spine (820a, 820b, 822a, 822b, 824a, 824b). The different arrangements may also include any other way in which the electrodes (830) may be positioned and/or oriented on one spine (820a, 820b, 822a, 822b, 824a, 824b) relative to the other electrodes (830) on the same spine (820a, 820b, 822a, 822b, 824a, 824b); and how that relative positioning and/or orientation may vary from one spine (820a, 820b, 822a, 822b, 824a, 824b) to another spine (820a, 820b, 822a, 822b, 824a, 824b).

### I. Ninth Example of Asymmetric Electrode Arrangement

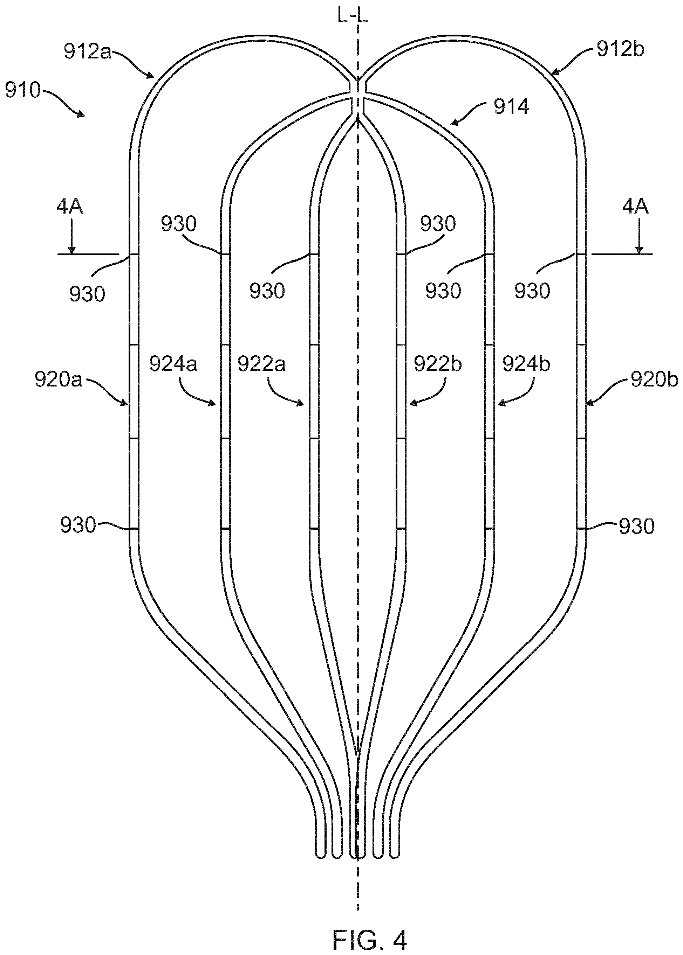

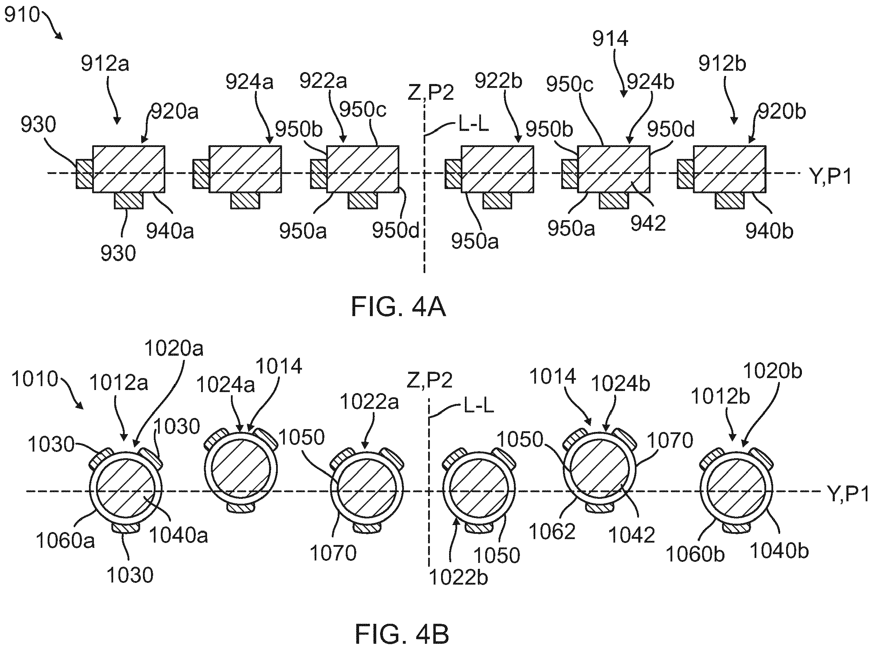

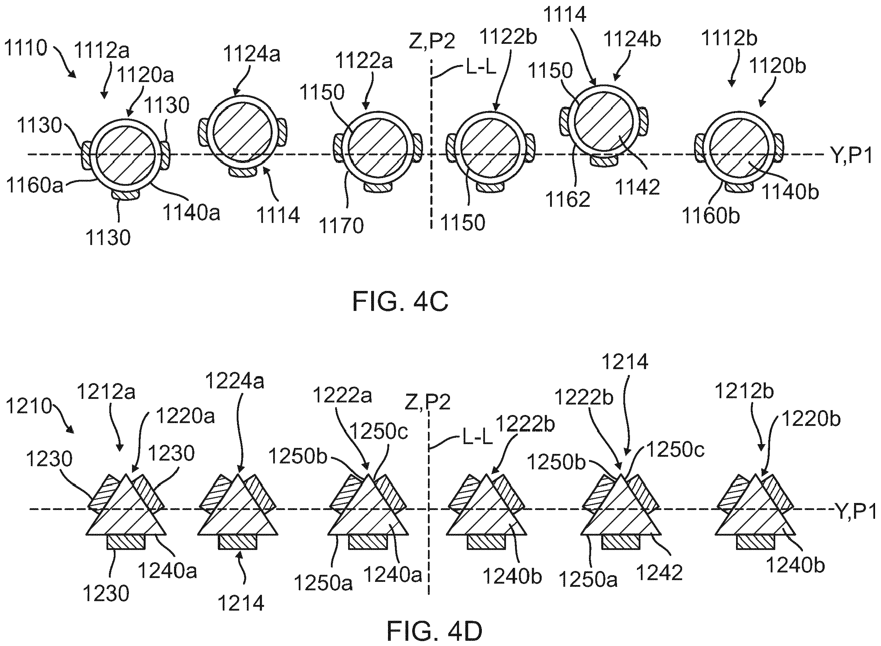

FIGS. 4-4A depict another example of an end effector (910) that may be readily incorporated into catheter (14) in place of distal tip (28). End effector (910) of the present example includes first and second outer loop members (912a, 912b) and an inner loop member (914), each extending distally from a base member or shaft (not shown), which itself extends along a longitudinal axis (L-L). Loop members (912a, 912b, 914) may also be referred to as paddles, loops, and/or electrode loop assemblies.

In the example shown, first and second outer loop members (912a, 912b) are disposed on either side of longitudinal axis (L-L), at least partially radially outwardly of third or central loop member (914). More particularly, outer loop members (912a, 912b) each include a corresponding outermost spine member (920a, 920b) and a corresponding innermost spine member (922a, 922b), while inner loop member (914) includes a pair of intermediate spine members (924a, 924b). Spine members (920a, 920b, 922a, 922b, 924a, 924b) may also be referred to as spines. In this regard, outermost spines (920a, 920b) are positioned radially outwardly of intermediate spines (924a, 924b) with respect to longitudinal axis (L-L), and innermost spines (922a, 922b) are positioned radially inwardly of intermediate spines (924a, 924b) with respect to longitudinal axis (L-L), at least within the frame of reference of FIG. 2. It will be appreciated that first outermost spine member (920a) is integral with first innermost spine member (922a) as part of first outer loop (912a) and that second outermost spine member (920b) is integral with second innermost spine member (922b) as part of second outer loop (912b). Similarly, first intermediate spine member (924a) is integral with second intermediate spine member (924b) as part of inner loop (914).

As best shown in FIG. 4A, spine members (920a, 920b, 922a, 922b, 924a, 924b) are generally arranged along a Y-axis that is orthogonal to longitudinal axis (L-L) such that end effector (910) may generally reside within a first plane (P1) defined by the Y-axis and longitudinal axis (L-L) and may therefore be generally planar. First outermost spine member (920a), first intermediate spine member (924a), and first innermost spine member (922a) are arranged on a first side of a second plane (P2) defined by longitudinal axis (L-L) and a Z-axis that is orthogonal to both the Y-axis and longitudinal axis (L-L); and second outermost spine member (920b), second intermediate spine member (924b), and second innermost spine member (922b) are arranged on a second side of the second plane (P2).

Loop members (912a, 912b, 914) also each include a corresponding plurality of sensing electrodes (930) secured to the respective spine members (920a, 920b, 922a, 922b, 924a, 924b) and arranged asymmetrically relative to the first plane (P1). Electrodes (930) may be configured to provide electrophysiology (EP) mapping, such as to identify tissue regions that should be targeted for ablation. For example, electrodes (930) may monitor electrical signals emanating from conductive endocardial tissues to pinpoint the location of aberrant conductive tissue sites that are responsible for an arrhythmia. By way of example only, electrodes (930) may be configured and operable in accordance with at least some of the teachings of U.S. Pub. No. 2020/0345262, entitled "Mapping Grid with High Density Electrode Array," published November 5, 2020, the disclosure of which is incorporated by reference herein, in its entirety.

In the example shown, each loop member (912a, 912b, 914) includes a corresponding structural member (940a, 940b, 942). In this regard, each outer loop member (912a, 912b) has a corresponding elongated structural member (940a, 940b) extending continuously through the length of the respective outer loop member (912a, 912b) to and from the base member, while inner loop member (914) has an elongated structural member (942) extending continuously through the length of inner loop member (914) to and from the base member. Structural members (940a, 940b, 942) may also be referred to as loop structural members, spine frames, or frames. Structural members (940a, 940b, 942) can each include a biocompatible metal such as stainless steel, cobalt chromium, or nitinol.

As best shown in FIG. 4A, each structural member (940a, 940b, 942) has four generally flat surfaces (950a, 950b, 950c, 950d) including a first generally flat surface (950a) oriented generally parallel to the first plane (P1), a second generally flat surface (950b) oriented generally orthogonal to the first plane (P1), a third generally flat surface (950c) oriented generally parallel to the first plane (P1), and a fourth generally flat surface (950d) oriented generally orthogonal to the first plane (P1), such that each structural member (940a, 940b, 942) of the present example has a generally rectangular cross-sectional shape. In the example shown, first and third surfaces (950a, 950c) each have a same length greater than that of second and fourth surfaces (950b, 950d). It will be appreciated that each structural member (940a, 940b, 942) may have any other suitable cross-sectional shape and/or may have any suitable number of different cross-sectional shapes that transition along a length of the structural member (940a, 940b, 942). For example, each structural member (940a, 940b, 942) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (912a, 912b, 914) may further include a corresponding cover (not shown), with the corresponding structural member (940a, 940b, 942) underlying the corresponding cover and with the corresponding plurality of electrodes (930) disposed on an outer surface of the corresponding cover (or otherwise exposed relative to the cover). Such covers may each be electrically insulative, and/or may each include a polymeric material such as polyurethane, for example.

As noted above, electrodes (930) of each loop member (912a, 912b, 914) are arranged asymmetrically relative to the first plane (P1) defined by end effector (910). In this regard, electrodes (930) of the present example are disposed only on first and second surfaces (950a, 950b) of structural members (940a, 940b, 942) of loop members (912a, 912b, 914), and are not disposed on third and four surfaces (950c, 950d) of structural members (940a, 940b, 942) of loop members (912a, 912b, 914). As shown, each electrode (930) disposed on a first surface (950a) of a structural member (940a, 940b, 942) is positioned at a same height along longitudinal axis (L-L) as a corresponding, adjacent electrode (930) disposed on the second surface (950b) of the same structural member (940a, 940b, 942), such that electrodes (930) may be arranged in adjacent groups of two. Each adjacent group of two electrodes (930) is asymmetric relative to the first plane (P1) defined by end effector (910), with the electrode (930) on first surface (950a) of each group being completely disposed on a first side of the first plane (P1) and the electrode (930) on second surface (950b) of each group spanning both the first and second sides of the first plane (P1).

While the arrangements of electrodes (930) are shown as being the same for each spine member (920a, 920b, 922a, 922b, 924a, 924b), it will be appreciated that electrodes (930) may be oriented in various different arrangements for each spine member (920a, 920b, 922a, 922b, 924a, 924b). For example, electrodes (930) may be oriented in a first arrangement on outermost spines (920a, 920b), in a second arrangement on innermost spines (922a, 922b), and in a third arrangement on intermediate spines (924a, 924b). It will be appreciated that the different arrangements of electrodes (930) may include the spacing between electrodes (930) on one spine (920a, 920b, 922a, 922b, 924a, 924b) differing from the spacing between electrodes (930) on another spine (920a, 920b, 922a, 922b, 924a, 924b). In addition, or in the alternative, the different arrangements may include the angular orientation of the electrodes (930) on one spine (920a, 920b, 922a, 922b, 924a, 924b) differing from the angular orientation of the electrodes (930) on another spine (920a, 920b, 922a, 922b, 924a, 924b). The different arrangements may also include any other way in which the electrodes (930) may be positioned and/or oriented on one spine (920a, 920b, 922a, 922b, 924a, 924b) relative to the other electrodes (930) on the same spine (920a, 920b, 922a, 922b, 924a, 924b); and how that relative positioning and/or orientation may vary from one spine (920a, 920b, 922a, 922b, 924a, 924b) to another spine (920a, 920b, 922a, 922b, 924a, 924b).

### J. Tenth Example of Asymmetric Electrode Arrangement

FIG. 4B depicts another example of an end effector (1010) that may be readily incorporated into catheter (14) in place of distal tip (28). End effector (1010) of the present example is similar to end effector (910) described above, except as otherwise described below. In this regard, end effector (1010) includes first and second outer loop members (1012a, 1012b) and an inner loop member (1014), each extending distally from a base member or shaft (not shown), which itself extends along a longitudinal axis (L-L). Outer loop members (1012a, 1012b) each include a corresponding outermost spine member (1020a, 1020b) and a corresponding innermost spine member (1022a, 1022b), while inner loop member (1014) includes a pair of intermediate spine members (1024a, 1024b).

As shown, spine members (1020a, 1020b, 1022a, 1022b, 1024a, 1024b) are generally arranged along a Y-axis that is orthogonal to longitudinal axis (L-L) such that end effector (1010) may generally reside within a first plane (P1) defined by the Y-axis and longitudinal axis (L-L) and may therefore be generally planar. First outermost spine member (1020a), first intermediate spine member (1024a), and first innermost spine member (1022a) are arranged on a first side of a second plane (P2) defined by longitudinal axis (L-L) and a Z-axis that is orthogonal to both the Y-axis and longitudinal axis (L-L); and second outermost spine member (1020b), second intermediate spine member (1024b), and second innermost spine member (1022b) are arranged on a second side of the second plane (P2).

Loop members (1012a, 1012b, 1014) also each include a corresponding plurality of sensing electrodes (1030) secured to the respective spine members (1020a, 1020b, 1022a, 1022b, 1024a, 1024b) and arranged asymmetrically relative to the first plane (P1). In the example shown, each loop member (1012a, 1012b, 1014) includes a corresponding structural member (1040a, 1040b, 1042), each having a generally cylindrical surface (1050) such that each structural member (1040a, 1040b, 1042) of the present example has a generally circular cross-sectional shape. It will be appreciated that each structural member (1040a, 1040b, 1042) may have any other suitable cross-sectional shape and/or may have any suitable number of different cross-sectional shapes that transition along a length of the structural member (1040a, 1040b, 1042). For example, each structural member (1040a, 1040b, 1042) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (1012a, 1012b, 1014) further includes a corresponding cover (1060a, 1060b, 1062), with the corresponding structural member (1040a, 1040b, 1042) underlying the corresponding cover (1060a, 1060b, 1062) and with the corresponding plurality of electrodes (1030) disposed on an outer surface (1070) of the corresponding cover (1060a, 1060b, 1062). Covers (1060a, 1060b, 1062) may each be electrically insulative, and/or may each include a polymeric material such as polyurethane, for example.

As noted above, electrodes (1030) of each loop member (1012a, 1012b, 1014) are arranged asymmetrically relative to the first plane (P1) defined by end effector (1010). In this regard, electrodes (1030) of the present example are circumferentially disposed on surfaces (1070) of covers (1060a, 1060b, 1062) of loop members (1012a, 1012b, 1014) at non-uniform intervals. As shown, each electrode (1030) disposed on a surface (1070) of a cover (1060a, 1060b, 1062) is positioned at a same height along longitudinal axis (L-L) as two corresponding, adjacent electrodes (1030) disposed on the surface (1070) of the same cover (1060a, 1060b, 1062), such that electrodes (1030) may be arranged in adjacent groups of three. Each adjacent group of three electrodes (1030) is asymmetric relative to the first plane (P1) defined by end effector (1010), with one electrode (1030) of each group being completely disposed on a first side of the first plane (P1) and the other two electrodes (1030) of each group being completely disposed on a second side of the first plane (P1).

While the arrangements of electrodes (1030) are shown as being the same for each spine member (1020a, 1020b, 1022a, 1022b, 1024a, 1024b), it will be appreciated that electrodes (1030) may be oriented in various different arrangements for each spine member (1020a, 1020b, 1022a, 1022b, 1024a, 1024b). For example, electrodes (1030) may be oriented in a first arrangement on outermost spines (1020a, 1020b), in a second arrangement on innermost spines (1022a, 1022b), and in a third arrangement on intermediate spines (1024a, 1024b). It will be appreciated that the different arrangements of electrodes (1030) may include the spacing between electrodes (1030) on one spine (1020a, 1020b, 1022a, 1022b, 1024a, 1024b) differing from the spacing between electrodes (1030) on another spine (1020a, 1020b, 1022a, 1022b, 1024a, 1024b). In addition, or in the alternative, the different arrangements may include the angular orientation of the electrodes (1030) on one spine (1020a, 1020b, 1022a, 1022b, 1024a, 1024b) differing from the angular orientation of the electrodes (1030) on another spine (1020a, 1020b, 1022a, 1022b, 1024a, 1024b). The different arrangements may also include any other way in which the electrodes (1030) may be positioned and/or oriented on one spine (1020a, 1020b, 1022a, 1022b, 1024a, 1024b) relative to the other electrodes (1030) on the same spine (1020a, 1020b, 1022a, 1022b, 1024a, 1024b); and how that relative positioning and/or orientation may vary from one spine (1020a, 1020b, 1022a, 1022b, 1024a, 1024b) to another spine (1020a, 1020b, 1022a, 1022b, 1024a, 1024b).

### K. Eleventh Example of Asymmetric Electrode Arrangement

FIG. 4C depicts another example of an end effector (1110) that may be readily incorporated into catheter (14) in place of distal tip (28). End effector (1110) of the present example is similar to end effector (910) described above, except as otherwise described below. In this regard, end effector (1110) includes first and second outer loop members (1112a, 1112b) and an inner loop member (1114), each extending distally from a base member or shaft (not shown), which itself extends along a longitudinal axis (L-L). Outer loop members (1112a, 1112b) each include a corresponding outermost spine member (1120a, 1120b) and a corresponding innermost spine member (1122a, 1122b), while inner loop member (1114) includes a pair of intermediate spine members (1124a, 1124b).

As shown, spine members (1120a, 1120b, 1122a, 1122b, 1124a, 1124b) are generally arranged along a Y-axis that is orthogonal to longitudinal axis (L-L) such that end effector (1110) may generally reside within a first plane (P1) defined by the Y-axis and longitudinal axis (L-L) and may therefore be generally planar. First outermost spine member (1120a), first intermediate spine member (1124a), and first innermost spine member (1122a) are arranged on a first side of a second plane (P2) defined by longitudinal axis (L-L) and a Z-axis that is orthogonal to both the Y-axis and longitudinal axis (L-L); and second outermost spine member (1120b), second intermediate spine member (1124b), and second innermost spine member (1122b) are arranged on a second side of the second plane (P2).

Loop members (1112a, 1112b, 1114) also each include a corresponding plurality of sensing electrodes (1130) secured to the respective spine members (1120a, 1120b, 1122a, 1122b, 1124a, 1124b) and arranged asymmetrically relative to the first plane (P1). In the example shown, each loop member (1112a, 1112b, 1114) includes a corresponding structural member (1140a, 1140b, 1142), each having a generally cylindrical surface (1150) such that each structural member (1140a, 1140b, 1142) of the present example has a generally circular cross-sectional shape. It will be appreciated that each structural member (1140a, 1140b, 1142) may have any other suitable cross-sectional shape and/or may have any suitable number of different cross-sectional shapes that transition along a length of the structural member (1140a, 1140b, 1142). For example, each structural member (1140a, 1140b, 1142) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In the example shown, each loop member (1112a, 1112b, 1114) further includes a corresponding cover (1160a, 1160b, 1162), with the corresponding structural member (1140a, 1140b, 1142) underlying the corresponding cover (1160a, 1160b, 1162) and with the corresponding plurality of electrodes (1130) disposed on an outer surface (1170) of the corresponding cover (1160a, 1160b, 1162). Covers (1160a, 1160b, 1162) may each be electrically insulative, and/or may each include a polymeric material such as polyurethane, for example.

As noted above, electrodes (1130) of each loop member (1112a, 1112b, 1114) are arranged asymmetrically relative to the first plane (P1) defined by end effector (1110). In this regard, electrodes (1130) of the present example are circumferentially disposed on surfaces (1170) of covers (1160a, 1160b, 1162) of loop members (1112a, 1112b, 1114) at non-uniform intervals. As shown, each electrode (1130) disposed on a surface (1170) of a cover (1160a, 1160b, 1162) is positioned at a same height along longitudinal axis (L-L) as two corresponding, adjacent electrodes (1130) disposed on the surface (1170) of the same cover (1160a, 1160b, 1162), such that electrodes (1130) may be arranged in adjacent groups of three. Each adjacent group of three electrodes (1130) is asymmetric relative to the first plane (P1) defined by end effector (1110), with one electrode (1130) of each group being at least partially disposed on a first side of the first plane (P1) and the other two electrodes (1130) of each group being at least partially disposed on a second side of the first plane (P1).

While the arrangements of electrodes (1130) are shown as being the same for each spine member (1120a, 1120b, 1122a, 1122b, 1124a, 1124b), it will be appreciated that electrodes (1130) may be oriented in various different arrangements for each spine member (1120a, 1120b, 1122a, 1122b, 1124a, 1124b). For example, electrodes (1130) may be oriented in a first arrangement on outermost spines (1120a, 1120b), in a second arrangement on innermost spines (1122a, 1122b), and in a third arrangement on intermediate spines (1124a, 1124b). It will be appreciated that the different arrangements of electrodes (1130) may include the spacing between electrodes (1130) on one spine (1120a, 1120b, 1122a, 1122b, 1124a, 1124b) differing from the spacing between electrodes (1130) on another spine (1120a, 1120b, 1122a, 1122b, 1124a, 1124b). In addition, or in the alternative, the different arrangements may include the angular orientation of the electrodes (1130) on one spine (1120a, 1120b, 1122a, 1122b, 1124a, 1124b) differing from the angular orientation of the electrodes (1130) on another spine (1120a, 1120b, 1122a, 1122b, 1124a, 1124b). The different arrangements may also include any other way in which the electrodes (1130) may be positioned and/or oriented on one spine (1120a, 1120b, 1122a, 1122b, 1124a, 1124b) relative to the other electrodes (1130) on the same spine (1120a, 1120b, 1122a, 1122b, 1124a, 1124b); and how that relative positioning and/or orientation may vary from one spine (1120a, 1120b, 1122a, 1122b, 1124a, 1124b) to another spine (1120a, 1120b, 1122a, 1122b, 1124a, 1124b).

### L. Twelfth Example of Asymmetric Electrode Arrangement

FIG. 4D depicts another example of an end effector (1210) that may be readily incorporated into catheter (14) in place of distal tip (28). End effector (1210) of the present example is similar to end effector (910) described above, except as otherwise described below. In this regard, end effector (1210) includes first and second outer loop members (1212a, 1212b) and an inner loop member (1214), each extending distally from a base member or shaft (not shown), which itself extends along a longitudinal axis (L-L). Outer loop members (1212a, 1212b) each include a corresponding outermost spine member (1220a, 1220b) and a corresponding innermost spine member (1222a, 1222b), while inner loop member (1214) includes a pair of intermediate spine members (1224a, 1224b).

As shown, spine members (1220a, 1220b, 1222a, 1222b, 1224a, 1224b) are generally arranged along a Y-axis that is orthogonal to longitudinal axis (L-L) such that end effector (1210) may generally reside within a first plane (P1) defined by the Y-axis and longitudinal axis (L-L) and may therefore be generally planar. First outermost spine member (1220a), first intermediate spine member (1224a), and first innermost spine member (1222a) are arranged on a first side of a second plane (P2) defined by longitudinal axis (L-L) and a Z-axis that is orthogonal to both the Y-axis and longitudinal axis (L-L); and second outermost spine member (1220b), second intermediate spine member (1224b), and second innermost spine member (1222b) are arranged on a second side of the second plane (P2).

Loop members (1212a, 1212b, 1214) also each include a corresponding plurality of sensing electrodes (1230) secured to the respective spine members (1220a, 1220b, 1222a, 1222b, 1224a, 1224b) and arranged asymmetrically relative to the first plane (P1). In the example shown, each loop member (1212a, 1212b, 1214) includes a corresponding structural member (1240a, 1240b, 1242), each having three generally flat surfaces (1250a, 1250b, 1250c) including a first generally flat surface (1250a) oriented generally parallel to the first plane (P1), a second generally flat surface (1250b) oriented generally obliquely to the first plane (P1), and a third generally flat surface (1250c) oriented generally obliquely to the first plane (P1), such that each structural member (1240a, 1240b, 1242) of the present example has a generally triangular cross-sectional shape. In the example shown, first, second, and third surfaces (1250a, 1250b, 1250c) each have a same length. It will be appreciated that each structural member (1240a, 1240b, 1242) may have any other suitable cross-sectional shape and/or may have any suitable number of different cross-sectional shapes that transition along a length of the structural member (1240a, 1240b, 1242). For example, each structural member (1240a, 1240b, 1242) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (1212a, 1212b, 1214) may further include a corresponding cover (not shown), with the corresponding structural member (1240a, 1240b, 1242) underlying the corresponding cover and with the corresponding plurality of electrodes (1230) disposed on an outer surface of the corresponding cover (or otherwise exposed relative to the cover). Such covers may each be electrically insulative, and/or may each include a polymeric material such as polyurethane, for example.

As noted above, electrodes (1230) of each loop member (1212a, 1212b, 1214) are arranged asymmetrically relative to the first plane (P1) defined by end effector (1210). In this regard, electrodes (1230) of the present example are disposed on each surface (1250a, 1250b, 1250c) of structural members (1240a, 1240b, 1242) of loop members (1212a, 1212b, 1214). As shown, each electrode (1230) disposed on a first surface (1250a) of a structural member (1240a, 1240b, 1242) is positioned at a same height along longitudinal axis (L-L) as two corresponding, adjacent electrodes (1230) disposed on the second and third surfaces (1250b, 1250c) of the same structural member (1240a, 1240b, 1242), such that electrodes (1230) may be arranged in adjacent groups of three. Each adjacent group of three electrodes (1230) is asymmetric relative to the first plane (P1) defined by end effector (1210), with the electrode (1230) on first surface (1250a) of each group being completely disposed on a first side of the first plane (P1) and the other two electrodes (1230) on second and third surfaces (1250b, 1250c) of each group being at least partially disposed on a second side of the first plane (P1).

While the arrangements of electrodes (1230) are shown as being the same for each spine member (1220a, 1220b, 1222a, 1222b, 1224a, 1224b), it will be appreciated that electrodes (1230) may be oriented in various different arrangements for each spine member (1220a, 1220b, 1222a, 1222b, 1224a, 1224b). For example, electrodes (1230) may be oriented in a first arrangement on outermost spines (1220a, 1220b), in a second arrangement on innermost spines (1222a, 1222b), and in a third arrangement on intermediate spines (1224a, 1224b). It will be appreciated that the different arrangements of electrodes (1230) may include the spacing between electrodes (1230) on one spine (1220a, 1220b, 1222a, 1222b, 1224a, 1224b) differing from the spacing between electrodes (1230) on another spine (1220a, 1220b, 1222a, 1222b, 1224a, 1224b). In addition, or in the alternative, the different arrangements may include the angular orientation of the electrodes (1230) on one spine (1220a, 1220b, 1222a, 1222b, 1224a, 1224b) differing from the angular orientation of the electrodes (1230) on another spine (1220a, 1220b, 1222a, 1222b, 1224a, 1224b). The different arrangements may also include any other way in which the electrodes (1230) may be positioned and/or oriented on one spine (1220a, 1220b, 1222a, 1222b, 1224a, 1224b) relative to the other electrodes (1230) on the same spine (1220a, 1220b, 1222a, 1222b, 1224a, 1224b); and how that relative positioning and/or orientation may vary from one spine (1220a, 1220b, 1222a, 1222b, 1224a, 1224b) to another spine (1220a, 1220b, 1222a, 1222b, 1224a, 1224b).

### M. Example of End Effector in Use

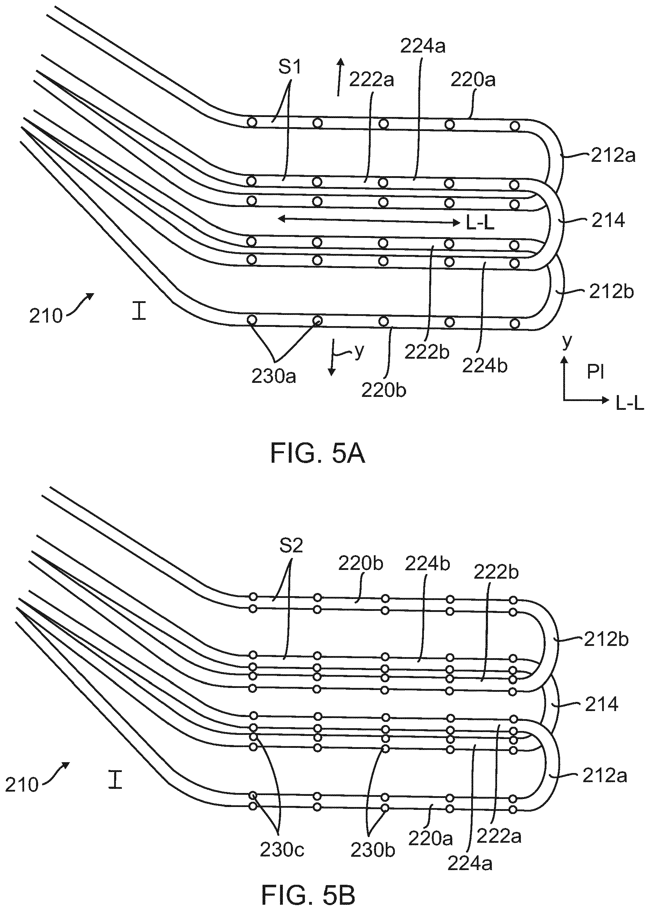

FIG. 5A depicts the end effector (210) of FIG. 2. As described above, the end effector (210) has the first and second outer loop members (212a, 212b) and the inner loop member (214), and the outer loop members (212a, 212b), The corresponding spine members (220a, 220b, 222a, 222b, 224a, 224b) are generally arranged along the Y-asis that is orthogonal to longitudinal axis (L-L) such that end effector (210) may generally reside within the first plane (P1) defined by the Y-axis and longitudinal axis (L-L) and may therefore be generally planar. As also described above, the loop members (212a, 212b, 214) also each includes a corresponding plurality of electrodes (230) arranged asymmetrically relative to the first plane (P1), where one electrode (230a) being disposed on a first side S1 of the spine members in the first plane (P1) and two other electrodes (230b, 230c) being disposed on a second side S2 of the spine members in the first plane (P1).

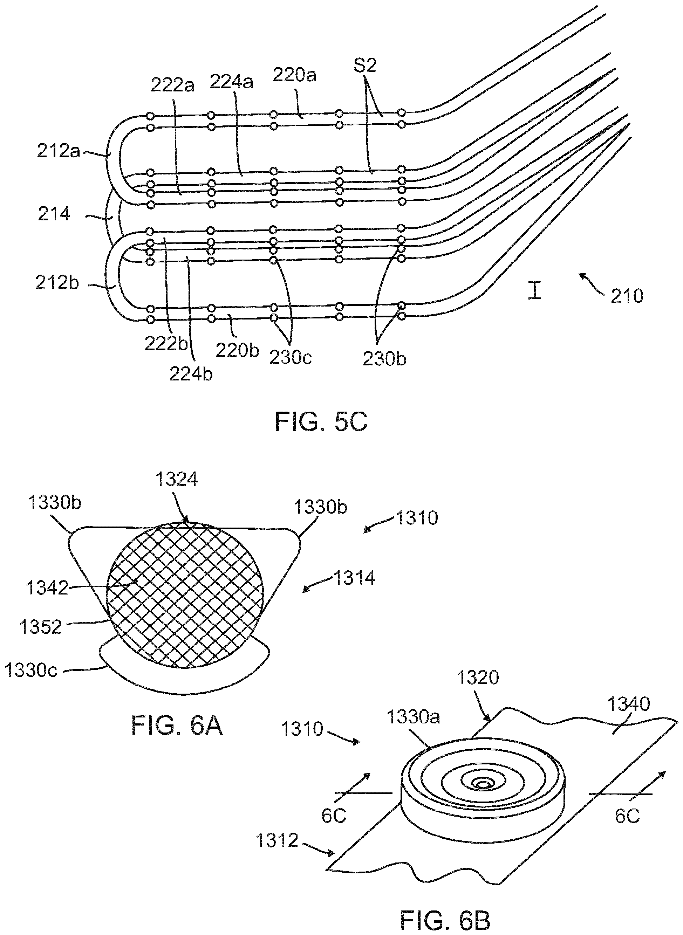

In laying the end effector (210), or sweeping the end effector (210) in one (or forward) direction, with the second side S2 facing tissue T, as depicted in FIG. 5A, the electrodes (230b, 230c) on the second side S1 are in contact with the tissue at respective locations or along respective tracks along the tissue. Subsequently, in flipping over the end effector (210) and laying the end effector, or sweeping the end effector in the one (or forward) direction, as depicted in FIG. 2B, or alternately, in laying the end effector (210) or sweeping the end effector (210) in an opposite (or backward) direction, with the first side S1 facing the tissue T, as depicted in FIG. 5C, the electrodes (230a) are now in contact with the tissue at their respective locations or tracks which are different and distinct from the respective locations and tracks of the electrodes (230b, 230c) on the second side S2. Advantageously, by arranging the electrodes (230a) and the electrodes (230b, 230c) asymmetrically relative to the plane P1, the end effector conveniently provides different contact locations between the electrodes and tissue for improved mapping and ablation, wherein either sets of locations and tracks can be readily accessed and used by merely flipping the end effector or laying or sweeping in the opposition direction.

It is understood that the use and advantages described above may be applied to any of the asymmetric arrangements of electrodes described herein.

### N. Thirteenth Example of Asymmetric Electrode Arrangement

FIGS. 6A-6C depict another example of an end effector (1310) that may be readily incorporated into catheter (14) in place of distal tip (28). End effector (1310) of the present example includes first and second outer loop members (1312) (one shown) and an inner loop member (1314), each extending distally from a base member or shaft (not shown), which itself extends along a longitudinal axis. Loop members (1312, 1314) may also be referred to as paddles, loops, and/or electrode loop assemblies. Outer loop members (1312) each include a corresponding pair of spine members (1320) (one shown), while inner loop member (1314) includes a pair of intermediate spine members (1324) (one shown).

Spine members (1320, 1324) may be generally arranged along a Y-axis that is orthogonal to the longitudinal axis such that end effector (1310) may generally reside within a plane defined by the Y-axis and longitudinal axis and may therefore be generally planar.

Loop members (1312, 1314) also each include a corresponding plurality of sensing electrodes (1330a, 1330b, 1330c) secured to the respective spine members (1320, 1324) and arranged asymmetrically relative to the plane. In the example shown, each loop member (1312, 1314) includes a corresponding structural member (1340, 1342), with the structural member (1340) of each outer loop member (1312) having four generally flat surfaces (1350a, 1350b, 1350c, 1350d) including a first generally flat surface (1350a) oriented generally parallel to the plane, a second generally flat surface (1350b) oriented generally orthogonal to the plane, a third generally flat surface (1350c) oriented generally parallel to the plane, and a fourth generally flat surface (1350d) oriented generally orthogonal to the plane, such that each structural member (1340) of the present example has a generally rectangular cross-sectional shape; while structural member (1342) of inner loop member (1314) has a generally cylindrical surface (1352) such that structural member (1342) of the present example has a generally circular cross-sectional shape. In the example shown, first and third surfaces (1350a, 1350c) each have a same length greater than that of second and fourth surfaces (1350b, 1350d). It will be appreciated that each structural member (1340, 1342) may have any other suitable cross-sectional shape and/or may have any suitable number of different cross-sectional shapes that transition along a length of the structural member (1340, 1342). For example, each structural member (1340, 1342) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

In some versions, each loop member (1312, 1314) may further include a corresponding cover (not shown), with the corresponding structural member (1340, 1342) underlying the corresponding cover and with the corresponding plurality of electrodes (1330a, 1330b, 1330c) disposed on an outer surface of the corresponding cover (or otherwise exposed relative to the cover). Such covers may each be electrically insulative, and/or may each include a polymeric material such as polyurethane, for example.

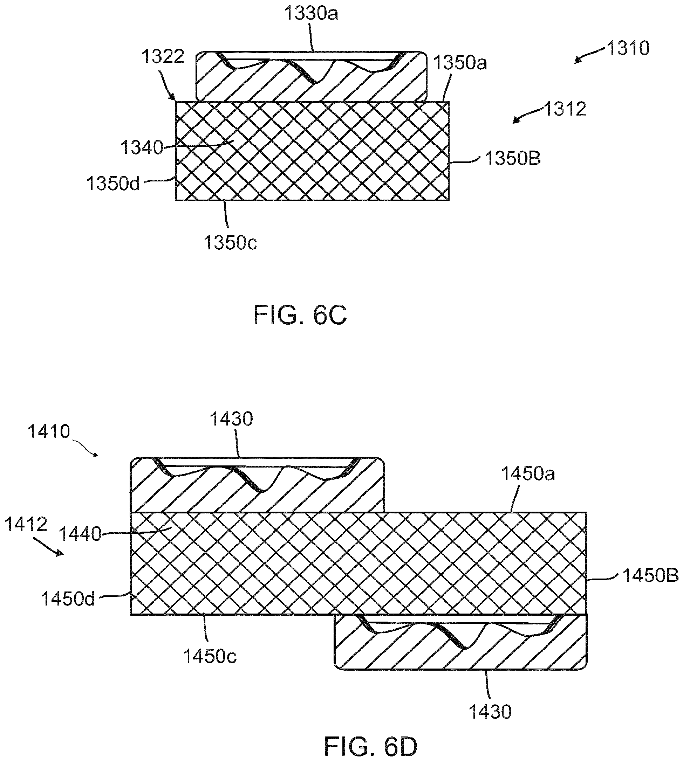

As noted above, electrodes (1330a, 1330b, 1330c) of each loop member (1312, 1314) are arranged asymmetrically relative to the plane defined by end effector (1310). In this regard, electrodes (1330a) of the present example are disposed on first surface (1350a) of each structural member (1340) of outer loop members (1312). As shown in FIGS. 6B and 6C, each electrode (1330a) disposed on a first surface (1350a) of a structural member (1340) is positioned at a height along the longitudinal axis at which no corresponding electrode (1330a) is disposed on the third surface (1350c) of the same structural member (1340) to provide an asymmetric arrangement of electrodes (1330a). As shown, each electrode (1330a) may itself be shaped to be asymmetric relative to another plane that extends through the electrode (1330a) and that is parallel to the plane defined by end effector (1310). In addition, or alternatively, electrodes (1330b, 1330c) of the present example are circumferentially disposed on surface (1352) of structural member (1342) of inner loop member (1314), with electrodes (1330b) having a first configuration and with electrodes (1330c) having a second configuration different from the first configuration. For example, electrodes (1330b) may each be generally triangular, while electrodes (1330c) may each be generally arcuate. As shown in FIG. 6A, each electrode (1330c) disposed on surface (1352) of structural member (1342) is positioned at a same height along the longitudinal axis as two corresponding, adjacent electrodes (1330b) disposed on the surface (1352) of structural member (1342), such that electrodes (1330b, 1330c) may be arranged in adjacent groups of three. Each adjacent group of three electrodes (1330b, 1330c) is asymmetric relative to the plane defined by end effector (1310), at least by virtue of the different configuration of each electrode (1330c) relative to the corresponding electrodes (1330b).

### O. Fourteenth Example of Asymmetric Electrode Arrangement

FIG. 6D depicts another example of an end effector (1410) that may be readily incorporated into catheter (14) in place of distal tip (28). End effector (1410) of the present example is similar to end effector (1310) described above, except as otherwise described below. In this regard, end effector (1410) includes first and second outer loop members (1412) (one shown) including a corresponding pair of spine members (1420) (one shown).

Spine members (1420) may be generally arranged along a Y-axis that is orthogonal to the longitudinal axis such that end effector (1410) may generally reside within a plane defined by the Y-axis and longitudinal axis and may therefore be generally planar.

Loop members (1412) also each include a corresponding plurality of sensing electrodes (1430) secured to the respective spine members (1420) and arranged asymmetrically relative to the plane defined by end effector (1410). In the example shown, each loop member (1412) includes a corresponding structural member (1440) having four generally flat surfaces (1450a, 1450b, 1450c, 1450d) including a first generally flat surface (1450a) oriented generally parallel to the plane, a second generally flat surface (1450b) oriented generally orthogonal to the plane, a third generally flat surface (1450c) oriented generally parallel to the plane, and a fourth generally flat surface (1450d) oriented generally orthogonal to the plane, such that each structural member (1440) of the present example has a generally rectangular cross-sectional shape. In the example shown, first and third surfaces (1450a, 1450c) each have a same length greater than that of second and fourth surfaces (1450b, 1450d). It will be appreciated that each structural member (1440) may have any other suitable cross-sectional shape and/or may have any suitable number of different cross-sectional shapes that transition along a length of the structural member (1440). For example, each structural member (1440) may have any one or more of a symmetrical shape, an asymmetrical shape, a polygonal shape, a triangular shape (e.g., Reuleaux triangle), a rectangular shape, a square shape, a pentagonal shape, an octagonal shape, a circular shape, a multi-curved shape, and/or an elliptical shape (e.g., irregular ellipse).

As noted above, electrodes (1430) of each loop member (1412) are arranged asymmetrically relative to the plane defined by end effector (1410). In this regard, electrodes (1430) of the present example are disposed on first and third surfaces (1450a, 1450c) of each structural member (1440) of outer loop members (1412). As shown, each electrode (1430) disposed on a first surface (1450a) of a structural member (1440) is positioned at a same height along the longitudinal axis as a corresponding, adjacent electrode (1430) disposed on the third surface (1450c) of the same structural member (1440), such that electrodes (1430) may be arranged in adjacent groups of two. Each adjacent group of two electrodes (1430) is asymmetric relative to the plane defined by end effector (1410), with the electrode (1430) on first surface (1450a) of each group being offset from the adjacent electrode (1430) on the third surface (1450b) along the Y-axis. As shown, each electrode (1430) may itself be shaped to be asymmetric relative to another plane that extends through the electrode (1430) and that is parallel to the plane defined by end effector (1410).

### III. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A catheter for electrophysiology applications, comprising: (a) a shaft extending along a longitudinal axis to a distal end; and (b) an end effector coupled to the distal end of the shaft, the end effector including: (i) a plurality of loop members, each loop member of the plurality of loop members including a corresponding pair of spines, the spines of each loop member of the plurality of loop members being arranged along a transverse axis that is perpendicular to the longitudinal axis, the transverse axis and the longitudinal axis collectively defining a plane, and (ii) a plurality of electrodes affixed to the plurality of loop members, the plurality of electrodes being arranged asymmetrically relative to the plane.

### Example 2

The catheter of Example 1, the plurality of loop members including three loop members.

### Example 3

The catheter of Example 2, the plurality of loop members including a pair of outer loop members and an inner loop member positioned radially inwardly of the pair of outer loop members relative to the longitudinal axis.

### Example 4

The catheter of Example 2, the plurality of loop members including a pair of inner loop members and an outer loop member positioned radially outwardly of the pair of inner loop members relative to the longitudinal axis.

### Example 5

The catheter of any of Examples 1 through 4, each loop member of the plurality of loop members including an elongate structural member.

### Example 6

The catheter of Example 5, each electrode of the plurality of electrodes being affixed to the elongate structural member of the corresponding loop member of the plurality of loop members.

### Example 7

The catheter of any of Examples 5 through 6, each loop member of the plurality of loop members including a cover disposed about the corresponding elongate structural member.

### Example 8

The catheter of Example 7, each electrode of the plurality of electrodes being affixed to the cover of the corresponding loop member of the plurality of loop members.

### Example 9

The catheter of any of Examples 1 through 8, each of the spines having a rectangular cross-sectional shape.

### Example 10

The catheter of Example 9, each of the spines having a pair of flat surfaces oriented parallel to each other, the plurality of electrodes being arranged on only one of the pair of flat surfaces of each of the spines.

### Example 11

The catheter of Example 9, each of the spines having: (A) a first flat surface oriented parallel to the plane, (B) a second flat surface oriented orthogonal to the plane, (C) a third flat surface oriented parallel to the plane, and (D) a fourth flat surface oriented orthogonal to the plane, the plurality of electrodes being arranged on only the first and second flat surfaces of each of the spines.

### Example 12

The catheter of any of Examples 1 through 8, each of the spines having a triangular cross-sectional shape.

### Example 13

The catheter of Example 12, each of the spines having three flat surfaces, the plurality of electrodes being arranged on each of the three flat surfaces of each of the spines.

### Example 14

The catheter of any of Examples 1 through 8, each of the spines having a circular cross-sectional shape.

### Example 15

The catheter of Example 14, each of the spines having a cylindrical surface, the plurality of electrodes being arranged on the cylindrical surface of each of the spines at non-uniform intervals.

### Example 16

The catheter of any of Examples 1 through 8, each of the spines having at least one polygonal cross-sectional shape with a plurality of surfaces.

### Example 17

The catheter of Example 16, the at least one polygonal cross-sectional shape including at least one of a pentagon or an octagon.

### Example 18

The catheter of any of Examples 16 through 17, the plurality of surfaces being arranged symmetrically relative to the plane.

### Example 19

The catheter of any of Examples 16 through 17, the plurality of surfaces being arranged asymmetrically relative to the plane.

### Example 20

The catheter of any of Examples 16 through 19, the at least one polygonal cross-sectional shape including a plurality of polygonal cross-sectional shapes provided along a length of the respective spine.

### Example 21

The catheter of Example 20, the plurality of polygonal cross-sectional shapes including a pentagon, a rectangle, and a triangle.

### Example 22

The catheter of any of Examples 1 through 8, each of the spines having at least one multi-curved cross-sectional shape with a plurality of curves.

### Example 23

The catheter of Example 22, the at least one multi-curved cross-sectional shape including at least one of an irregular ellipse or a Reuleaux triangle.

### Example 24

The catheter of any of Examples 22 through 23, the plurality of curves being arranged symmetrically relative to the plane.

### Example 25

The catheter of any of Examples 22 through 23, the plurality of curves being arranged asymmetrically relative to the plane.

### Example 26

The catheter of any of Examples 1 through 8, each of the spines having a plurality of cross-sectional shapes provided along a length of the respective spine.

### Example 27

The catheter of Example 26, the plurality of cross-sectional shapes including a square, an ellipse, and a circle.

### Example 28

An end effector of a catheter, the end effector comprising: (a) a plurality of loop members, each loop member of the plurality of loop members including a corresponding pair of spines, the spines of each loop member of the plurality of loop members including at least a portion extending parallel to a longitudinal axis, the spines of each loop member of the plurality of loop members being arranged along a transverse axis that is perpendicular to the longitudinal axis, the transverse axis and the longitudinal axis collectively defining a plane; and (b) a plurality of electrodes affixed to the plurality of loop members, the plurality of electrodes being arranged asymmetrically relative to the plane.

### Example 29

The end effector of Example 28, each of the spines having at least one of a rectangular cross-sectional shape, a triangular cross-sectional shape, or a circular cross-sectional shape.

### Example 30

An end effector of a catheter, the end effector comprising: (a) a central loop member extending along a longitudinal axis; (b) a first side loop member at least partially disposed on a first side of the longitudinal axis; (c) a second side loop member at least partially disposed on a second side of the longitudinal axis, the second side loop member, the first side loop member, and the central loop member being arranged along a transverse axis that is perpendicular to the longitudinal axis, the transverse axis and the longitudinal axis collectively defining a plane; and (d) a plurality of electrodes, each of the plurality of electrodes being affixed to a corresponding one of the central loop member, the first side loop member, or the second side loop member, the plurality of electrodes being arranged asymmetrically relative to the plane.

### Example 31

The end effector of Example 30, the central loop member being positioned radially inwardly of each of the first and second side loop members relative to the longitudinal axis.

### Example 32

The end effector of Example 30, the central loop member being positioned radially outwardly of each of the first and second side loop members relative to the longitudinal axis.

### IV. Miscellaneous

It is noted that the electrodes described and illustrated herein are not limited to mapping (i.e., sensing signals or recording signals) but can be used to deliver energy such as RF (alternating cycle) or IRE (DC pulses) in bipolar or unipolar mode alone or in combination with the mapping or sensing function. In the application for IRE, and by way of example only, the electrodes may be configured to deliver at least 900V per electrode with a current of at least 20 amperes over a number of pulses sufficient to cause cell apoptosis.

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A catheter for electrophysiology applications, comprising:
(a) a shaft extending along a longitudinal axis to a distal end; and
(b) an end effector coupled to the distal end of the shaft, the end effector including:
(i) a plurality of loop members, each loop member of the plurality of loop members including a corresponding pair of spines, the spines of each loop member of the plurality of loop members being arranged along a transverse axis that is perpendicular to the longitudinal axis, the transverse axis and the longitudinal axis collectively defining a plane, and
(ii) a plurality of electrodes affixed to the plurality of loop members, the plurality of electrodes being arranged asymmetrically relative to the plane.

2. The catheter of claim 1, the plurality of loop members including three loop members.

3. The catheter of claim 2, the plurality of loop members including (ii) a pair of outer loop members and an inner loop member positioned radially inwardly of the pair of outer loop members relative to the longitudinal axis, or (ii) a pair of inner loop members and an outer loop member positioned radially outwardly of the pair of inner loop members relative to the longitudinal axis.

4. The catheter of claim 1, each loop member of the plurality of loop members including an elongate structural member.

5. The catheter of claim 4, each electrode of the plurality of electrodes being affixed to the elongate structural member of the corresponding loop member of the plurality of loop members.

6. The catheter of claim 4, each loop member of the plurality of loop members including a cover disposed about the corresponding elongate structural member, optionally wherein each electrode of the plurality of electrodes being affixed to the cover of the corresponding loop member of the plurality of loop members.

7. The catheter of claim 1, each of the spines is configured with a rectangular cross-sectional shape.

8. The catheter of claim 7, wherein (i) each of the spines is configured with a pair of flat surfaces oriented parallel to each other, the plurality of electrodes being arranged on only one of the pair of flat surfaces of each of the spines, or (ii) each of the spines includes:
(A) a first flat surface oriented parallel to the plane,
(B) a second flat surface oriented orthogonal to the plane,
(C) a third flat surface oriented parallel to the plane, and
(D) a fourth flat surface oriented orthogonal to the plane,
the plurality of electrodes being arranged on only the first and second flat surfaces of each of the spines.

9. The catheter of claim 1, wherein each of the spines is configured with a triangular cross-sectional shape, optionally wherein each of the spines is configured with three flat surfaces, the plurality of electrodes being arranged on each of the three flat surfaces of each of the spines.

10. The catheter of claim 1, wherein each of the spines is configured with a circular cross-sectional shape, optionally wherein each of the spines is configured with a cylindrical surface, the plurality of electrodes being arranged on the cylindrical surface of each of the spines at non-uniform intervals.

11. The catheter of claim 1, wherein each of the spines is configured with at least one polygonal cross-sectional shape with a plurality of surfaces.

12. The catheter of claim 11, the at least one polygonal cross-sectional shape including at least one of a pentagon or an octagon.

13. The catheter of claim 11, the plurality of surfaces being arranged symmetrically relative to the plane, or asymmetrically relative to the plane.

14. The catheter of claim 11, the at least one polygonal cross-sectional shape including a plurality of polygonal cross-sectional shapes provided along a length of the respective spine, optionally wherein the plurality of polygonal cross-sectional shapes includes a pentagon, a rectangle, and a triangle.

15. The catheter of claim 1, wherein each of the spines is configured with at least one multi-curved cross-sectional shape with a plurality of curves.

16. The catheter of claim 15, the at least one multi-curved cross-sectional shape including at least one of an irregular ellipse or a Reuleaux triangle.

17. The catheter of claim 15, the plurality of curves being arranged symmetrically relative to the plane, or asymmetrically relative to the plane.

18. The catheter of claim 1, wherein each of the spines is configured with a plurality of cross-sectional shapes provided along a length of the respective spine, optionally wherein the plurality of cross-sectional shapes includes a square, an ellipse, and a circle.

19. An end effector of a catheter, the end effector comprising:
(a) a plurality of loop members, each loop member of the plurality of loop members including a corresponding pair of spines, the spines of each loop member of the plurality of loop members including at least a portion extending parallel to a longitudinal axis, the spines of each loop member of the plurality of loop members being arranged along a transverse axis that is perpendicular to the longitudinal axis, the transverse axis and the longitudinal axis collectively defining a plane; and
(b) a plurality of electrodes affixed to the plurality of loop members, the plurality of electrodes being arranged asymmetrically relative to the plane.

20. The end effector of claim 19, each of the spines is configured with at least one of a rectangular cross-sectional shape, a triangular cross-sectional shape, or a circular cross-sectional shape.

21. An end effector of a catheter, the end effector comprising:
(a) a central loop member extending along a longitudinal axis;
(b) a first side loop member at least partially disposed on a first side of the longitudinal axis;
(c) a second side loop member at least partially disposed on a second side of the longitudinal axis, the second side loop member, the first side loop member, and the central loop member being arranged along a transverse axis that is perpendicular to the longitudinal axis, the transverse axis and the longitudinal axis collectively defining a plane; and
(d) a plurality of electrodes, each of the plurality of electrodes being affixed to a corresponding one of the central loop member, the first side loop member, or the second side loop member, the plurality of electrodes being arranged asymmetrically relative to the plane.

22. The end effector of claim 21, the central loop member being positioned radially inwardly of each of the first and second side loop members relative to the longitudinal axis, or radially outwardly of each of the first and second side loop members relative to the longitudinal axis.

23. The end effector of claim 21, wherein an electrode of the plurality of electrodes is asymmetric (i) relative to the plane, or (ii) relative to a first plane that extends through the electrode and that is parallel to the plane defined by the end effector.
